# EUROPEAN PATENT SPECIFICATION

(11) **EP 4 059 546 B1**
(45) Date of publication and mention of the grant of the patent: **18.02.2026**
(21) Application number: 20887973.4
(22) Date of filing: 15.06.2020
(51) Int. Cl.: A61M 5/20, A61M 5/145, A61M 5/142

(54) **INJECTION PUMP**
EINSPRITZPUMPE
POMPE D'INJECTION

(30) Priority: 15.11.2019 CN 201911122416
(43) Date of publication of application: 21.09.2022
(73) Proprietor: Shenzhen Mindray Scientific Co., Ltd., Shenzhen, Guangdong 518057 (CN)
(72) Inventor: LIU, Huayong, Shenzhen, Guangdong 518107 (CN); ZHAO, Pengshi, Shenzhen, Guangdong 518107 (CN); WU, Dingwei, Shenzhen, Guangdong 518107 (CN); ZUO, Pengfei, Shenzhen, Guangdong 518107 (CN)
(74) Representative: KIPA AB
(86) International application number: PCT/CN2020/096219
(87) International publication number: WO 2021/093325

(56) References cited:
- CN-A- 1 490 060
- CN-A- 103 120 816
- CN-A- 103 120 816
- CN-A- 105 343 967
- CN-A- 105 363 101
- CN-A- 106 362 237
- CN-A- 106 860 976
- CN-A- 109 481 782
- CN-U- 203 954 331
- CN-U- 205 055 072
- CN-U- 205 055 072
- CN-U- 205 287 122
- CN-U- 205 964 619
- US-A- 3 456 649
- US-A1- 2007 100 283
- US-A1- 2010 160 861
- US-A1- 2017 326 293

## Description

### TECHNICAL FIELD

The disclosure relates to the technical field of medical devices, and more particularly to an injection pump.

### BACKGROUND

The injection pump refers to a device which is operable to push its piston for injection and infusion, thus realizing high-precision, stable and pulsatile liquid transmission. However, as there are many assemblies in the injection pump and their layout are unreasonable, the structure of the injection pump in the related technology is bulky and its volume is too large, which is not conducive to the miniaturization of the injection pump.

CN103120816A provides a syringe pump. The syringe pump includes housing, actuating device, driving device, circuit board module and battery modules. The housing includes a back cover and a middle plate, the back cover has relative opening and blind end, and the middle plate is fixed on the opening of the back cover. The blind end of the back cover is recessed to form the battery compartment for accommodating the battery modules to its opening direction. The actuating device, the driving device and the circuit board module are all contained in the back cover.

US20170326293 disclose an infusion apparatus for use with a syringe, where this apparatus includes a housing enclosing a drivetrain. The drivetrain includes a pinion, spur gears, and a worm gear. One spur gear is arranged to engage with the pinion and another spur gear is arranged to engage with the worm gear. The housing also includes a carriage movable with respect to the housing. A frame on the carriage receives a syringe plunger. A rack on the carriage engages with the pinion to move the carriage parallel to a longitudinal axis of the housing. A pusher assembly of the housing securely engages with the syringe plunger. A motor in the apparatus rotates a worm drive that meshes with the worm gear to drive the drivetrain. A trigger of the apparatus is configured to disengage the worm drive from the worm gear to allow free movement of the carriage relative to the housing.

US20070100283 discloses an external infusion device that infuses a fluid into an individual's body. The infusion device includes a housing, a reservoir, a drive system, a power supply, electrical elements, and a tab. The reservoir contains the fluid, and the drive system forces the fluid from the reservoir. The electrical elements control the power to the drive system to regulate the rate that fluid is forced from the reservoir. The tab mates with the housing, and contains at least one electrical element. The tab is removable, and may be replaced with a different tab. The different tab may change the rate fluid is forced from the reservoir. A tab may be removed from one external infusion device and installed in a different external infusion device. The tab may be limited to use in a predetermined number of external infusion devices and may include a power supply.

CN205055072U discloses a syringe pump that is from locking -type syringe presser device. The syringe pump includes a casing that has an inner chamber, a potentiometre accommodating in the inner chamber, a potentiometre circuit board that links with the potentiometre, a potentiometre connector one end of which is connected to the potentiometer, a locking piece connected at the other end of the potentiometre connector, a supporting block arranged at one side of the locking piece, a pivot shaft connected with the potentiometre connector and passing through the locking piece and the supporting block, a fixed pin cooperated with the pivot shaft, and a spring between the potentiometre connector and the spring. The locking piece performs a reciprocating motion along the pivot shaft, and the potentiometre connector rotates around the pivot shaft.

### SUMMARY

Based on these, an embodiment of this disclosure expects to provide an injection pump with a relatively compact structure.

**In** order to achieve the above purpose, one aspect of an embodiment of this disclosure provides an injection pump which is used together with a syringe, including:
a housing, which includes a bottom housing and a cover plate;
a syringe installation assembly, wherein the syringe installation assembly, the bottom housing and the cover plate enclose an inner cavity of the housing; the syringe installation assembly comprises an installation base and a clamping shank, and the clamping shank rotates relative to the installation base to clamp or release the syringe;
a push-pull box assembly, which is arranged outside the housing and faces an end of the syringe installation assembly;
a pump body device, which is arranged inside the inner cavity and is located at a side of the inner cavity, which is adjacent to the syringe installation assembly; wherein the pump body device includes a base body, and a slide assembly, a connection shaft, a transmission assembly and a drive mechanism, which are arranged at the base body; wherein one end of the connection shaft is connected with the slide assembly, and the other end of the connection shaft, which is away from the slide assembly, is located outside the housing and connected with the push-pull box assembly; wherein the drive mechanism is arranged at a side of the base body, which is away from the syringe installation assembly, and the drive mechanism is in drive connection with the transmission assembly, and the transmission assembly is in transmission connection with the slide assembly;
a battery module, which is arranged inside the inner cavity, wherein the battery module is located at the same side of the base body where the drive mechanism is arranged;
a power module, which is arranged inside the inner cavity, along a transverse direction which is perpendicular to a length direction of the pump body device; wherein the power module is arranged at the same side of the base body where the battery module is arranged and the power module is arranged side-by-side to the battery module, wherein one of the battery module and the power module is located along an extension line of an output shaft of the drive mechanism;
a display screen assembly, which is arranged at a side of the syringe installation assembly, which is away from the pump body device; wherein the display screen assembly covers the clamping shank of the syringe installation assembly.

Another aspect of an embodiment of this disclosure provides an injection pump which is used together with a syringe, including:
a housing, which includes a bottom housing and a cover plate;
a syringe installation assembly, which includes an installation base and a clamping shank, wherein the installation base, the bottom housing and the cover plate enclose an inner cavity of the housing; wherein a clamping channel and a clamping shank installation space are formed on a side wall of the installation base, which is away from the inner cavity; wherein the clamping shank installation space is communicated with the clamping channel from a side of the clamping channel, and the clamping shank installation space is arranged at an end of the installation base and communicated with outside; wherein the clamping shank is arranged inside the clamping shank installation space, and the clamping shank rotates relative to the installation base to clamp or release the syringe;
a push-pull box assembly, which is arranged outside the housing and faces an end of the clamping channel, which is adjacent to the clamping shank;
a pump body device, which is arranged inside the inner cavity and is located at a side of the inner cavity, which is adjacent to the syringe installation assembly; wherein the pump body device includes a base body, and a slide assembly, a connection shaft, a transmission assembly and a drive mechanism which are arranged at the base body; wherein the base body has an accommodation cavity, which is located at a side of the base body, which is away from the syringe installation assembly; wherein the slide assembly includes two guide rods, which are arranged at intervals and in parallel inside the accommodation cavity, and a slide block, which is attached to the two guide rods; wherein the connection shaft is a hollow shaft which is arranged between the two guide rods, one end of the hollow shaft is connected with the slide block, and the other end of the hollow shaft, which is away from the slide block, is located outside the housing and connected with the push-pull box assembly; wherein the drive mechanism is arranged inside the accommodation cavity and is located at a side of the accommodation cavity, which is away from the push-pull box assembly; wherein the transmission assembly includes a gear mechanism, which is in drive connection with the drive mechanism, and a screw rod, which is fixedly connected with the gear mechanism, wherein the screw rod is in transmission connection with the slide assembly and is inserted inside the hollow shaft;
a battery module, which is arranged inside the inner cavity and located at an extension line of an output shaft of the drive mechanism;
a power module, which is arranged inside the inner cavity, along a transverse direction, which is perpendicular to a length direction of the pump body device; wherein the power module and the battery module are arranged side-by-side at a same side of the base body;
a display screen assembly, which is arranged at a side of the syringe installation assembly, which is away from the pump body device and covers the syringe installation assembly.

The injection pump according to the embodiment of this disclosure can greatly save the installation space in the housing, make the overall structure of the injection pump more compact, thus being conducive to the miniaturization of the injection pump, through arranging the drive mechanism at the side of the base body, which is away from the syringe installation assembly, arranging the power module along a transverse direction which is perpendicular to a length direction of the pump body device and arranging one of the battery module and the power module at an extension line of an output shaft of the drive mechanism.

### BRIEF DESCRIPTION OF THE DRAWINGS

FIG. 1 is a structural block diagram of a pump body device of an injection pump according to an embodiment of this disclosure.
FIG.2 is a structural block diagram of the pump body device shown in FIG.1 from another perspective.
FIG.3 is a structural block diagram of the pump body device shown in FIG.1 from a further perspective.
FIG.4 is a sectional view of part structure of the pump body device shown in FIG.1.
FIG.5 is a structural diagram showing the cooperation between the spiral groove of the screw rod and the spiral tooth of the screw nut shown in FIG. 4.
FIG.6 is a structural diagram showing a coordination between a spiral groove of another screw rod and a spiral tooth of another screw nut.
FIG.7 is a structural diagram of the base body shown in FIG.1.
FIG. 8 is a structural diagram of the plunger assembly shown in FIG.1
FIG.9 is a structural block diagram of the plunger assembly shown in FIG.1 from another perspective.
FIG. 10 is an exploded view of the plunger assembly shown in FIG.8.
FIG. 11 is a structural diagram of the elongated potentiometer shown in FIG.1.
FIG. 12 is a structural diagram showing the cooperation between the pump body device, the push-pull box assembly and the cable shown in FIG.1.
FIG. 13 is a structural diagram showing the cooperation between the clamping claw of the push-pull box assembly and the relevant structures inside the box shown in FIG.12.
FIG. 14 is an exploded view of the push-pull box assembly shown in FIG.12.
FIG. 15 is a structural diagram showing the cooperation between the injection pump and the syringe according to an embodiment of this disclosure.
FIG. 16 is a structural diagram of the injection pump shown in FIG.15 after removing the cover plate.
FIG. 17 is a structural diagram of the injection pump shown in FIG.15 after removing the cover plate and display screen assembly.
FIG. 18 is a structural diagram of the syringe installation assembly shown in FIG.17.
FIG. 19 is a structural diagram of the syringe installation assembly shown in FIG.17 from another perspective.
FIG. 20 is a structural diagram showing the cooperation between the clamp shank, the rotation assembly, the second potentiometer and the coupler shown in FIG.19.
FIG. 21 is a structural diagram of the cooperation structure shown in FIG. 20 after removing the protective sleeve.

### DESCRIPTION OF THE PREFERRED EMBODIMENTS

It should be noted that the embodiments and the technical features in the embodiments in this disclosure can be combined with each other without conflict. The detailed description in the specific embodiment should be understood as an explanation of the purpose of this disclosure and should not be regarded as an improper restriction on this disclosure.

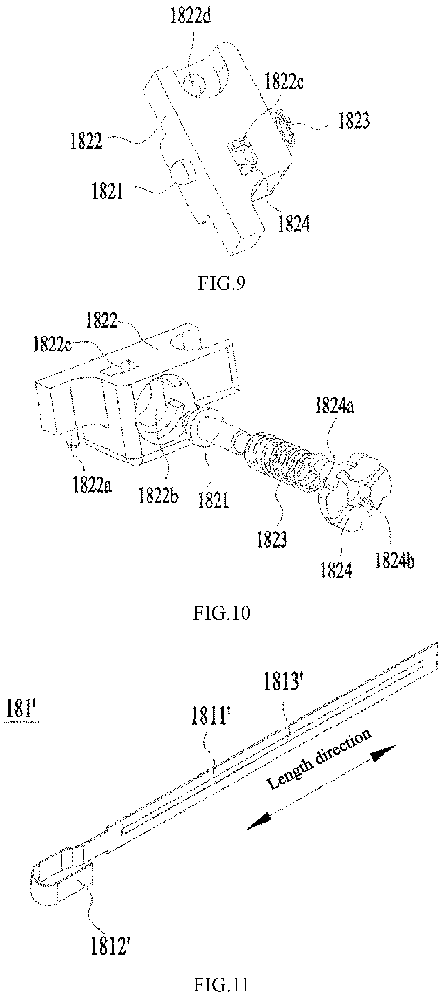

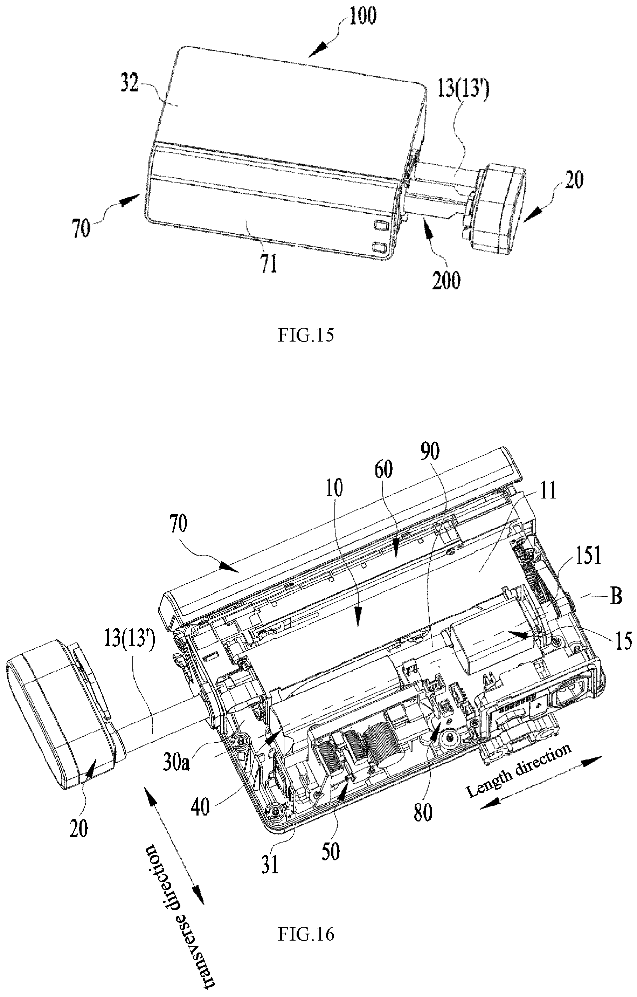

In the description of this disclosure, the orientation or position relationship related with "transverse direction" and "length direction" is based on the orientation or position relationship shown in FIG. **11** and FIG. 16. It should be understood that these orientation or position terms are only for the convenience of describing this disclosure and simplifying the description, rather than indicating or implying that, the device or element must have a specific orientation or position or must be constructed and operated in a specific orientation or position, so they cannot be understood as a limitation of this disclosure.

This disclosure has disclosed an injection pump which is used together with a syringe, for realizing high-precision, stable and pulsatile liquid transmission.

Referring to FIGS. 15 to 17, the injection pump 100 of this embodiment includes a housing 30, a syringe installation assembly 60, a push-pull box assembly 20, a pump body device 10, a battery module 40, a power module 50 and a display screen assembly 70. The housing 30 includes a bottom housing 31 and a cover plate 32. The syringe installation assembly 60, the bottom housing 31 and the cover plate 32 enclose an inner cavity 30a of the housing 30. The syringe installation assembly 60 is operable to fix a syringe 200. The push-pull box assembly 20 is arranged outside the housing 30 and faces one end of the syringe installation assembly 60. The push-pull box assembly 20 is operable to clamp a piston of the syringe 200 and realize liquid transmission by pushing the piston. The pump body device 10 is arranged inside the inner cavity 30a and is located at, a side of the inner cavity 30a, which is adjacent to the syringe installation assembly 60. The pump body device 10 includes a base body 11, a slide assembly 12, a connection shaft 13, a transmission assembly 14 and a drive mechanism 15. Wherein the slide assembly 12, connection shaft 13, transmission assembly 14 and drive mechanism 15 are arranged at the base body 11. One end of the connection shaft 13 is connected with the slide assembly 12, and the other end of the connection shaft 13, which is away from the slide assembly 12, is located outside the housing 30 and connected with the push-pull box assembly 20. The drive mechanism 15 is located at a side of the base body 11, which is away from the syringe installation assembly 60, and the drive mechanism 15 is in drive connection with the transmission assembly 14, and the transmission assembly 14 is in transmission connection with the slide assembly 12. Driven by the drive mechanism 15, the push-pull box assembly 20 can reciprocate relative to the base body 11 through the cooperation of the transmission assembly 14 and the slide assembly 12. The battery module 40 is arranged inside the inner cavity 30a, wherein the battery module 40is located at the same side of the base body 11 where the drive mechanism 15 is located. The power module 50 is arranged inside the inner cavity 30a, along a transverse direction which is perpendicular to a length direction of the pump body device 10, wherein the power module 50 and the battery module 40 are arranged side-by-side at the same side of the base body 11. One of the battery module 40 and the power module 50 is located at an extension line of an output shaft 151 of the drive mechanism 15 (i.e., the dotted line shown at B in FIG. 16). The display screen assembly 70 is arranged at a side of the syringe installation assembly 60, which is away from the pump body device 10. The display screen assembly 70 covers the syringe installation assembly 60 along the transverse direction which is perpendicular to the length direction of the pump body device 10.

Specifically, the present embodiment mainly arranges the drive mechanism 15 at the side of the base body 11, which is away from the push-pull box assembly 20, arranges the battery module 40 at the extension line of the output shaft 151 of the drive mechanism 15, arranges the power module 50 at the side of the battery module 40, which is away from the pump body device 10. Meanwhile, the overall shape of the power module 50 and the battery module 40 is an elongated strip. Accordingly, the length directions of the battery module 40 and the power module 50 are parallel to the length direction of the pump body device 10, while the transverse direction, which is perpendicular to the length direction of the pump body device 10, is the thickness direction of the battery module 40 and the power module 50. Thus, the pump body device 10, the battery module 40 and the power supply module 50 can be arranged more compactly. In addition, the arrangement of the battery module 40 at the extension line of the output shaft 151 of the drive mechanism 15 can also avoid the adverse effect of the heat generated by the drive mechanism 15 on the battery module 40. It can be understood that, in other embodiments, the drive mechanism 15 can also be arranged at one end of the base body 11, which is adjacent to the push-pull box assembly 20. In other embodiments, the power module 50 can also be arranged at the extension line of the output shaft 151 of the drive mechanism 15.

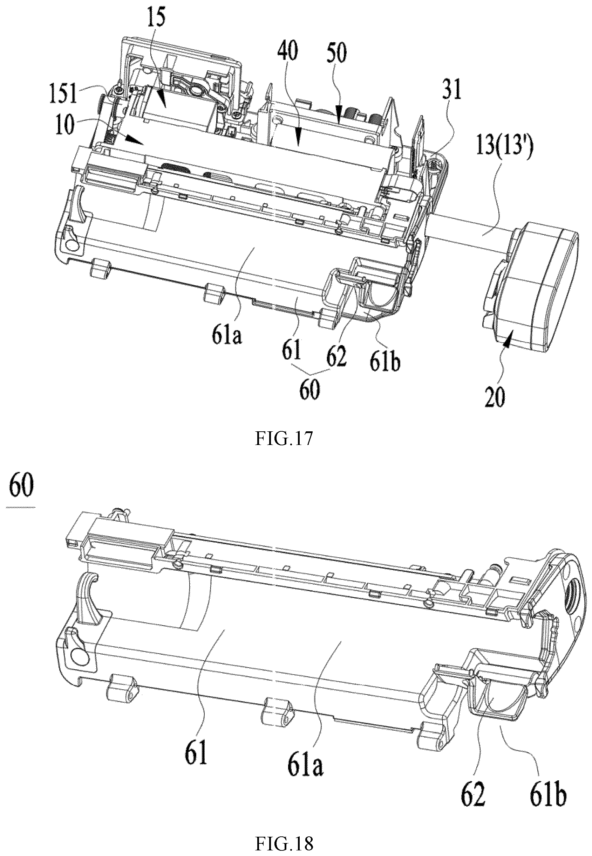

Referring to FIGS. 17 and 18, the syringe installation assembly 60 of this embodiment includes an installation base 61 and a clamping shank 62. The installation base 61, the bottom housing 31 and the cover plate 32 enclose the inner cavity 30a. A clamping channel 61a and a clamping shank installation space 61b, which is communicated with the clamping channel 61a from a side of the clamping channel 61a, are formed on a side wall of the installation base 61, which is away from the inner cavity 30a. The clamping shank installation space 61b is arranged at an end of the installation base 61, which is adjacent to the push-pull box assembly 20, and the clamping shank installation space 61b is communicated with outside. The clamping shank 62 is arranged inside the clamping shank installation space 61b, and the clamping shank 62 rotates relative to the installation base 61 to clamp or release the syringe 200.

In related technologies, the clamping shank is arranged at a side of the display screen assembly along the length direction of the display screen assembly. That is, the clamping shank is located outside an opening at one end of the clamping channel. When installing the syringe, it is necessary to pull out the clamping shank in the direction, which is perpendicular to the axis of the clamping channel (some clamping shanks can also rotate along their own axis after being pulled out), and then insert the syringe into the clamping channel, such that the clamping shank clamps the syringe through a reverse movement. This arrangement method requires a large installation space to install the clamping shank.

In this embodiment, the clamping shank 62 is arranged at a side of the clamping channel 61a and clamps or releases the syringe 200 by rotation. Since it is not necessary to pull out the clamping shank 62 in the direction which is perpendicular to the axis of the clamping channel 61a, this arrangement method can reduce the installation space of the clamping shank 62.

In addition, the purpose of communicating the clamping shank installation space 61b with the outside is to facilitate the user to operate the clamping shank 62. Specifically, when the user moves the clamping shank 62 toward the syringe 200, the clamping shank 62 clamps the syringe 200. When the user moves the clamping shank 62 away from the syringe 200, the clamping shank 62 releases the clamped syringe 200.

In this embodiment, the clamping shank installation space 61b is arranged at the side of the clamping channel 61a, which is adjacent to the bottom housing 31. In other embodiments, the clamping shank installation space 61b can also be arranged at the side of the clamping channel 61a, which is away from the bottom housing 31.

Further, referring to FIG. 15, the display screen assembly 70 covers the syringe installation assembly 60. That is, from the direction which faces a screen 71 of the display screen assembly 70, the display screen assembly 70 completely blocks the syringe installation assembly 60, but the display screen assembly 70 will not affect the normal use of the syringe installation assembly 60.

In related technologies, in order to avoid the interference between the outwardly pulled clamping shank and the display screen assembly, the clamping shank can only be installed at the side of the display screen assembly along the length direction of the display screen assembly, that is, the clamping shank actually occupies the space of the display screen assembly, so that the installation area for the display screen assembly is relatively small, and the screen area in the display screen assembly is relatively small.

In this embodiment, since it is not necessary to pull out the clamping shank 62 in the direction, which is perpendicular to the axis of the clamping channel 61a, this installation method can also avoid the interference between the clamping shank 62 and the display screen assembly 70, so that the display screen assembly 70 can cover the whole syringe installation assembly 60 which includes the clamping shank 62, so as to increase the surface area of the screen 71 and improve the user experience.

In addition, it should be noted that the display screen assembly 70 of this embodiment is actually rotatably connected with the installation base 61, that is, the display screen assembly 70 is equivalent to a pump door which is arranged outside the clamping channel 61a. When the display screen assembly 70 rotates to a door opening position along the direction which is away from the clamping channel 61a, the clamping channel 61a is exposed. At this time, it is convenient to clamp or take out the syringe 200. When the display screen assembly 70 rotates to a door closing position in the direction which is adjacent to the clamping channel 61a, the display screen assembly 70 blocks the clamping channel 61a. Therefore, it can prevent dust from falling to the clamping shank 62 and syringe 200, making them clean, sanitary and beautiful. **In** addition, it can avoid touching the syringe 200 which is clamped inside the clamping channel 61a by mistake.

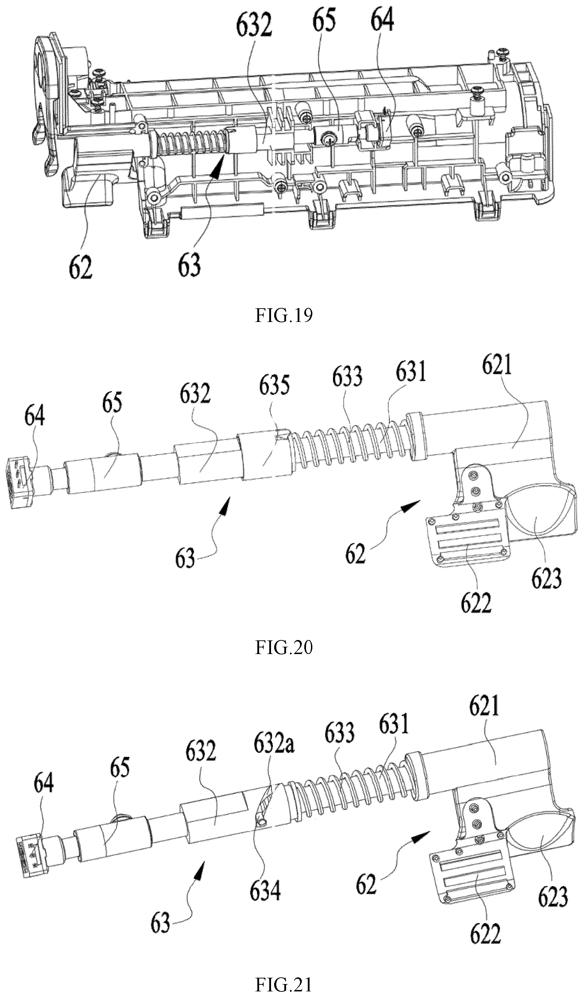

Referring to FIGS. 19 to 20, the syringe installation assembly 60 also includes a rotation assembly 63, and the clamping shank 62 is connected with the rotation assembly 63 to realize the rotation of the clamping shank 62.

Specifically, the clamping shank 62 of the present embodiment includes a connection part 621, a clamping part 622 and a pressure part 623, while the rotation assembly 63 includes a clamping shank shaft 631, a power transmission slide block 632, a compression spring 633, a pin shaft 634 and a protective sleeve 635.

The connection part 621 is connected with one end of the clamping shank shaft 631, and the other end of the clamping shank shaft 631 is rotatably connected with the installation base 61. The pressure part 623 drives the connection part 621 to rotate synchronously with the clamping shank shaft 631, to a clamping position or a release position. When the clamping part 622 is in the clamping position, the syringe 200 can be clamped, and when the clamping part 622 is in the release position, the syringe 200 can be released.

The power transmission slide block 632, the pin shaft 634 and at least a part of the clamping shank shaft 631 are arranged inside the installation base 61, wherein the power transmission slide block 632 is provided with a spiral groove 632a, and the installation base 61 positionally limits the rotation of the power transmission slide block 632. The pin shaft 634 is connected with the clamping shank shaft 631, and the pin shaft 634 is slidably matched with the spiral groove 632a. The compression spring 633 is sheathed outside the clamping shank shaft 631, and both ends of the compression spring 633 respectively abut against the power transmission slide block 632 and the connection part 621. The pin shaft 634 slides along the spiral groove 632a to push the power transmission slide block 632 to move axially to compress or release the compression spring 633.

Both ends of the spiral groove 632a are closed to prevent the pin shaft 634 from slipping out. One end of the spiral groove 632a, which is away from the connection part 621, is provided with a dead center. When the pin shaft 634 is located at the dead center, the clamping shank 62 can be maintained in the release position.

The protective sleeve 635 is detachably sheathed outside the power transmission slide block 632 and covers the spiral groove 632a. The clamping shank shaft 631 is provided with an installation hole, at least a part of the pin shaft 634 is installed inside the installation hole, and the protective sleeve 635 covers both ends of the pin shaft 634.

During usage, the pressure part 623 of the clamping shank 62 is pressed down to drive the clamping shank shaft 631 to rotate through the connection part 621, such that the pin shaft 634 rotates with the clamping shank shaft 631 to push the power transmission slide block 632 to move axially towards the clamping shank 62, thus squeezing the compression spring 633. When the space between the clamping part 622 of the clamping shank 62 and the clamping channel 61a is enough to place the syringe 200, the syringe 200 can be directly put into the clamping channel 61a and then the pressure part 623 is released. Alternatively, the pressure part 623 can be pressed until the pin shaft 634 enters the dead center position of the spiral groove 632a, and then the syringe 200 can be put into the clamping channel 61a. Then, the pressure part 623 is moved upward to separate the pin shaft 634 from the dead center position of the spiral groove 632a, such that the pressure part 623 is released. At this time, under the action of restoring force, the compression spring 633 pushes the power transmission slide block 632 to move axially away from the clamping shank 62, and the power transmission slide block 632 pushes the pin shaft 634 to rotate, the pin shaft 634 drives the clamping shank shaft 631 to rotate, thus driving the clamping part 622 to rotate in the direction towards the clamping channel 61a through the connection part 621 to clamp the syringe 200.

It can be understood that in other embodiments, a tension spring can also be used to replace the compression spring 633, or a torsion spring structure can be used to realize the rotation of the clamping shank 62. The clamping shank 62 can also be designed to first pull the clamping shank 62 out for a certain distance in the direction of the push-pull box assembly 20 along the axial direction of the clamping channel 61a, and then clamp the syringe 200 by rotating the clamping shank 62.

Further, the syringe installation assembly 60 of this embodiment also includes a second potentiometer 64 and a coupler 65. The second potentiometer 64 is connected with the clamping shank shaft 631 through the coupler 65. The second potentiometer 64 can detect the angle change of the rotated clamping shank shaft 631 and can determine the outer diameter of the corresponding syringe 200 through different angles according to the corresponding feedback algorithm. Further, the size and capacity of the syringe 200 held by the clamping shank 62 can be determined in the database.

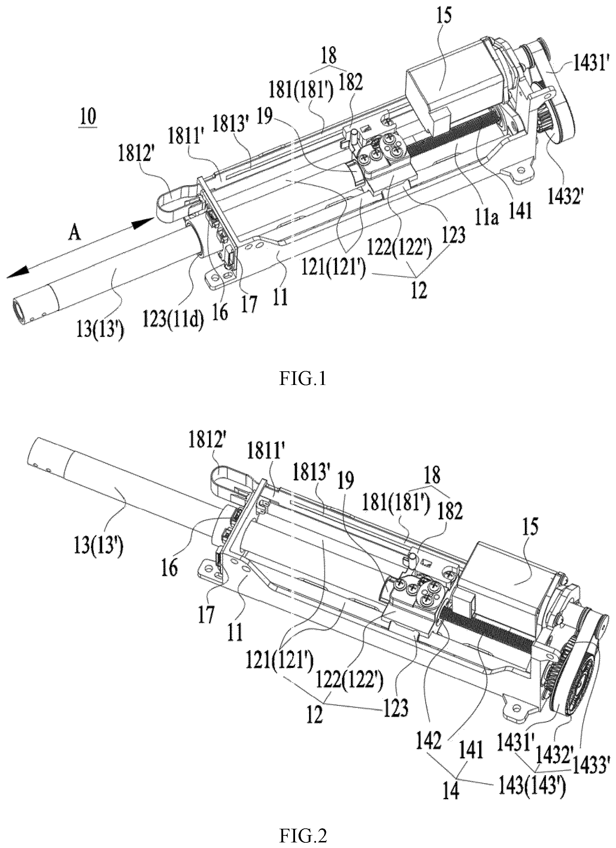

Referring to FIGS. 1 to 4, the connection shaft 13 of this embodiment is a hollow shaft 13', the slide assembly 12 includes a first slide member 121 which is arranged at the base body 11 and a second slide member 122 which is slidably connected with the first slide member 121, while the transmission assembly 14 includes a screw rod 141, which is in drive connection with the second slide member 122. The screw rod 141 is arranged in parallel and at intervals with the first slide member 121 and is inserted inside the hollow shaft 13'. Driven by the drive mechanism 15, the screw rod 141 can convert its own rotational motion into a linear motion of the second slide member 122, so that the second slide member 122 can slide relative to the first slide member 121, thus enabling the push-pull box assembly 20 to reciprocate relative to the base body 11.

In related technologies, the push-pull box assembly is usually connected with the second slide member through a connection shaft, and the connection shaft is arranged in parallel and spaced with the screw rod, so that the connection shaft and the screw rod need to occupy a large installation space in the base body, which makes the volume of the pump body device larger.

In this embodiment, the connection shaft 13 is designed as a hollow shaft 13 ', and the hollow shaft 13' is sheathed with the screw rod 141 and meanwhile connected with the second slide member 122. The screw rod 141 can rotate inside the hollow shaft 13' to convert its own rotational motion into the linear motion of the second slide member 122. The drive mechanism 15 may be a motor with a drive function, such as a stepping motor. Driven by the drive mechanism 15, the second slide member 122 can reciprocate in a straight line along the axial direction of the screw rod 141 (i.e., the direction shown by the double arrow at A in FIG. 1). Therefore, the second slide member 122 can drive the push-pull box assembly 20 which is connected with the hollow shaft 13' to reciprocate along the axial direction of the screw rod 141. Compared with the connection shaft and screw rod which are arranged in parallel and at intervals, the sheathed hollow shaft 13' and screw rod 141 can greatly save the installation space. Therefore, the structure of the pump body device 10 can be made more compact, and then the overall structure of the injection pump 100 can be made more compact. The term "reciprocate" described in this application refers to that when an injection is required, the push-pull box assembly 20 can push the syringe 200 to move in the injection direction under the driving of the second slide member 122. When the injection is completed and the syringe 200 is removed from the injection pump 100, the push-pull box assembly 20 can be driven to move in the opposite direction through the second slide member 122 immediately to reset the push-pull box assembly 20. Or the push-pull box assembly 20 is not reset immediately, however, when the next injection is required, the push-pull box assembly 20 is driven to move in the opposite direction through the second slide member 122 firstly. After the push-pull box assembly 20 is reset, the new syringe 200 is clamped. That is, the term "reciprocate" described in this application mainly refers to that the push-pull box assembly 20 can move in two opposite directions, but does not request that the push-pull box assembly 20 must move continuously between two opposite directions in a certain period of time. It can be understood that in other embodiments, instead of using the hollow shaft 13', an ordinary connection shaft 13 can be used, and the screw rod 141 can be arranged at intervals and in parallel with the connection shaft 13.

Referring to FIG. 1 to FIG. 3 and FIG. 7, the transmission assembly 14 of this embodiment also includes a screw nut 142 which is threaded with the screw rod 141. The screw rod 141 has a spiral groove 141a which has an arc normal section shape. The screw nut 142 has a spiral tooth 142a which is matched with the spiral groove 141a. The screw nut 142 is arranged at the second slide member 122. Though driving of the drive mechanism 15, the rotary motion of the screw rod 141 can be converted into the linear motion of the screw nut 142, so that the screw nut 142 can drive the second slide member 122 to slide relative to the first slide member 121.

Specifically, in related technologies, the injection pump generally uses the trapezoidal screw rod to convert its own rotation motion into the linear motion of the second slide member. However, the guide accuracy of the trapezoidal screw rod is not high, which will have an adverse impact on the use of the injection pump. In order to improve the guide accuracy, some injection pumps use the ball screw rod mechanism to replace the trapezoidal screw rod. However, on the one hand, the cost of the ball screw rod mechanism is high. On the other hand, as its structure is relatively complex, the volume of the ball screw rod mechanism is also relatively large, which needs to occupy a large installation space in the pump body device and is not conducive to the miniaturization of the injection pump.

This embodiment mainly improves the traditional screw rod and the screw nut which is matched with the screw rod. The normal section shape of the spiral groove 141a of the screw rod 141 is arc, and the tooth shape of the spiral tooth 142a of the screw rod 142 matches with the arc spiral groove 141a, that is, the tooth shape of the spiral tooth 142a of the screw rod 142 is actually arc. Compared with the trapezoidal screw rod, the guide accuracy of the arc screw rod 141 of this embodiment is higher can meet the use requirements of the injection pump 100. While compared with the ball screw rod mechanism, the arc screw rod 141 and screw nut 142 of this embodiment have lower cost and relatively small installation space, so is conducive to the miniaturization of the injection pump 100, while reducing the production cost of the injection pump 100

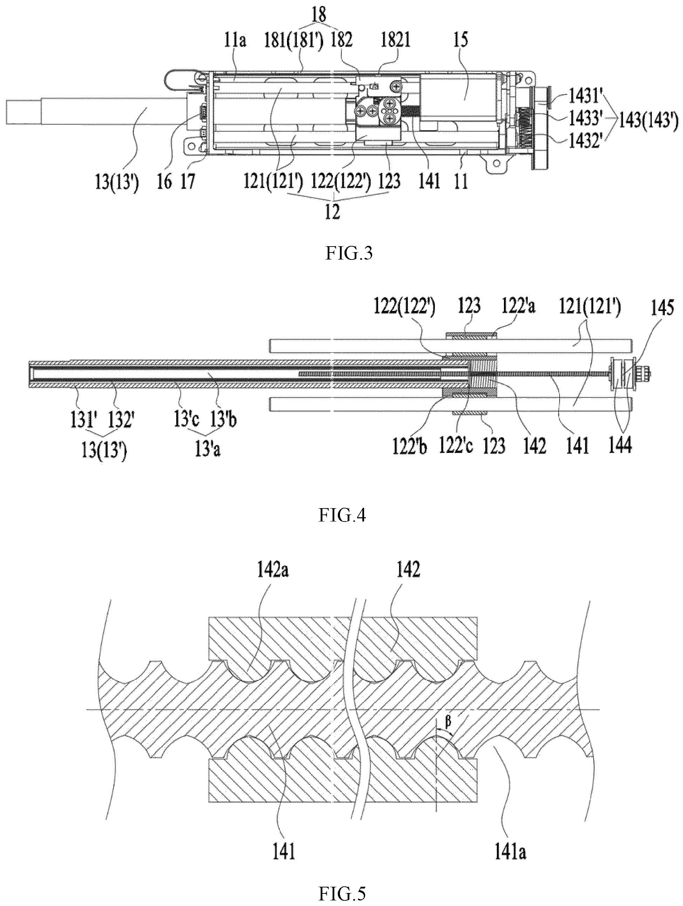

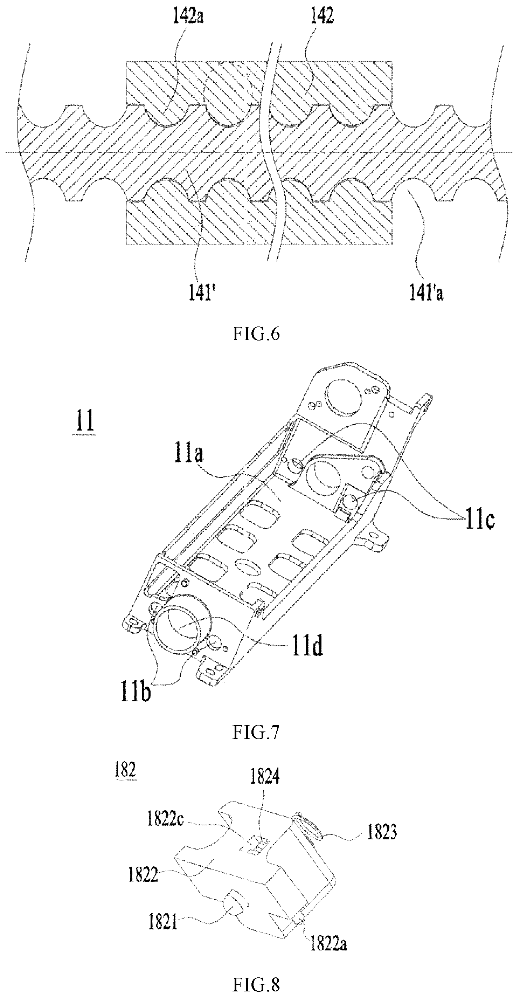

Referring to FIG. 5, the normal section shape of the spiral groove 141a of this embodiment is bi-arc, and the tooth shape of the spiral tooth 142a is semi-circular. Contact angle β between the spiral groove 141a and spiral tooth 142a can be arranged as needed. Preferably, the contact angle β between the spiral groove 141a and the spiral tooth 142a (the angle between the normal of the tangent point of the spiral groove 141a and the spiral tooth 142a, and the vertical line, which is perpendicular to the axis of the screw rod 141) is 45°. It can be understood that the spiral groove 141a can also be other arc structures. For example, referring to FIG. 6, the normal section shape of the spiral groove 141'a of the screw rod 141' can also be oval. In other embodiments, the normal section shape of the spiral groove 141a can also be a single arc shape, and so on. According to the different normal section shapes of the spiral groove 141a, which are matched with the spiral tooth 142a, the tooth shape of the spiral tooth 142a can also be other arc structures. For example, the tooth form of the spiral tooth 142a can also be a single arc, oval, double circular arc and so on, besides semicircle.

Referring to FIG. 4, a third installation hole 122'c which penetrates through the second slide member 122 is formed on the second slide member 122 of the present embodiment. The screw nut 142 is detachably arranged in the third installation hole 122'c.

Specifically, the screw nut 142 is arranged inside the third installation hole 122'c and is threaded with the second slide member 122. At the same time, one end of the connection shaft 13 which is adjacent to the screw nut 142 is also inserted into the third installation hole 122'c. That is, the screw nut 142 is arranged inside the second slide member 122, which can save the installation space of the screw nut 142, and the screw nut 142 is threaded with the second slide member 122, which can facilitate the disassembly and assembly of the second slide member 122 and the screw nut 142. In other embodiments, the second slide member 122 and the screw nut 142 can also be integrally formed, which is equivalent to directly machining a screw hole with spiral teeth 142a at the second slide member 122. In other embodiments, a trapezoidal screw rod or a ball screw rod mechanism may also be used.

It should be noted that in related technologies, an injection pump adopts the method of matching the screw rod and the clutch nut to realize the transmission of the second slide member, and the screw rod and the clutch nut can be separated. During the use of this injection pump, through the cooperation of the drive mechanism and the transmission assembly, it can only drive the push-pull box assembly to push the syringe to move in the injection direction. When the injection is completed and the push-pull box assembly needs to be reset, the operator needs to manually operate, such as manually rotate, the push-pull box assembly, to separate the screw rod from the clutch nut, and then continue to manually pull the push-pull box assembly back in the opposite direction, that is, this kind of injection pump actually has a semi-manual operation mode. During injection, the push-pull box assembly is driven by the drive mechanism, while the push-pull box assembly is manually pulled back during reset.

The screw rod 141 and the screw nut 142 of the pump body device 10 of this embodiment cannot be separated. At the same time, driven by the drive mechanism 15, the screw nut 142 can reciprocate along the screw rod 141 in a straight line, so that no matter the movement towards the injection direction or its reset, of the push-pull box assembly 20, which is connected with the second slide member 122 through the hollow shaft 13', can be realized by electric control, which saves the trouble of manual operation. In addition, when resetting the push-pull box assembly 20, since it is not necessary to separate the screw rod 141 and the screw nut 142 manually, it is not necessary to configure the corresponding mechanical structure for the separation of the screw rod 141 and the screw nut 142, so that the installation space can be further saved, which makes the structure of the transmission assembly 14 of the present embodiment simpler and the overall structure of the pump body device 10 more compact.

Referring to FIG. 4, the transmission assembly 14 of this embodiment also includes two bearings 144 which are arranged adjacent to the base body 11. One end of the screw rod 141, which is in drive connection with the drive mechanism 15, penetrates through and is arranged inside the two bearings 144. The other end of the screw rod 141, which is away from the bearing 144, is a free end.

In related technologies, most of the screw rods of the injection pump are supported by one bearing at one end, and the end of the screw rod, which is away from the bearing, is matched with a copper sleeve as the rotary support. However, the installation accuracy of this arrangement method is poor, and there is a risk of pump jamming. In the rotation process of screw rod, the end of the screw rod, which is away from the bearing, is noisy, and the copper sleeve is seriously worn, so there is a certain safety risk.

In this embodiment, the end of the screw rod 141, which is in the drive connection with the drive mechanism 15, is supported by two bearings 144. More specifically, the bearing 144 in this embodiment is a deep groove ball bearing, and a gasket 145 is sandwiched between the two deep groove ball bearings. The end of the screw rod 141, which is away from the bearing 144, is not provided with a support structure. Since the two bearings 144 can bear large axial force, the stability of high-speed movement can also be ensured when the end of the screw rod 141, which is away from the bearing 144, is not provided with a support structure. At the same time, since the end of the screw rod 141, which is away from the bearing 144 does not need to be provided with a support structure such as a copper sleeve, the screw rod 141 of this embodiment can reduce the motion noise during rotation, and there is no risk of pump jamming and wearing of the support structure, Thus, the safety of the injection pump 100 is greatly improved.

It can be understood that in other embodiments, the bearing 144 can also be an angular contact bearing, and the two angular contact bearings can be installed back-to-back DB or face-to-face DF. In other embodiments, only one bearing 144 may be provided at one end of the screw rod 141, or more than two bearings 144 may be provided, or one or more bearings 144 may be provided at both ends of the screw rod 141.

Please continue to refer to FIGS 1 to 4 and 7. The first slide member 121 of this embodiment is the guide rod 121', and the second slide member 122 is a slide block 122 which is penetrated by the guide rod 121', and the slide block 122'can slide along the guide rod 121'.

Specifically, the base body 11 of the present embodiment has an accommodation cavity 11a, and a first positioning hole 11b, a second positioning hole 11c and a position-limiting hole 11d, which are respectively communicated with the accommodation cavity 11a. The first positioning hole 11b is arranged at a side of the base body 11, which is adjacent to the push-pull box assembly 20. The second positioning hole 11c is arranged opposite to the first positioning hole 11b, one end of the guide rod 121' is inserted into the first positioning hole 11b, and the other end of the guide rod 121', which is away from the first positioning hole 11b, is inserted into the second positioning hole 11c. That is, the guide rod 121 'of this embodiment is inserted into and matched with the base body 11. Since two guide rods 121' are arranged in this embodiment, two first positioning holes 11b and two second positioning holes 11c are correspondingly arranged at the base body 11. The position-limiting hole 11d is on the same side as the first positioning hole 11b, and the hollow shaft 13' penetrates through the position-limiting hole 11d. Driven by the slide block 122', the hollow shaft 13' can reciprocate along the axis of the position-limiting hole 11d. The position-limiting hole 11d can guide the hollow shaft 13' to make the hollow shaft 13' more stable in the reciprocation process.

Further, a first installation hole 122'a and a second installation hole 122'b, which are arranged at intervals, are formed at the slide block 122'. The projection of the first installation hole 122'a along the axial direction is a closed hole, and the projection of the second installation hole 122'b along the axial direction is a semi-closed hole. One of the two guide rods 121' penetrates through the first installation hole 122'a, and the other one penetrates through the second installation hole 122'b. In other words, the slide assembly 12 of this embodiment is a cooperation structure of double guide rods and slide block 122'. At the same time, please refer to FIG. 4, the expression "projection of the first installation hole 122'a along the axial direction is a closed hole" in this embodiment mainly refers to that at least part of the side wall of the first installation hole 122'a is a ring-shaped closed structure, so that the guide rod 121' which penetrates through the first installation hole 122'a can be constrained radially. The expression "projection of the second installation hole 122'b along the axial direction is a semi-closed hole", mainly refers to that the side wall of the second installation hole 122'b has at least one notch which is arranged along the axial direction, so that the guide rod 121', which penetrates through the second installation hole 122'b, can have a certain offset in the radial direction. That is, using the second installation hole 122'b, a certain adjustment allowance can be reserved for the slide block 122' in the direction which is perpendicular to the guide rod 121' to ensure the guidance accuracy of the guide rod 121'. It can be understood that the number of guide rods 121' is not limited to two. In other embodiments, the number of guide rods 121' can be one or more than two. The section shape of the guide rod 121 'is not limited. For example, the section surface of the guide rod 121' can be circular, triangular, rectangular, special-shaped, etc., as long as the guide rod 12' can match with the slide block 122'.

In other embodiments, the first slide member 121 can also be a slide grove, while the second slide member 122 is a guide block which is matched with the slide grove. Alternatively, the first slide member 121 can also be a guide rail, and the second slide member 122 is a guide base which is provided with guide wheels, as long as the first slide member 121 and the second slide member 122 can cooperate with each other.

In related technologies, in order to ensure the smooth movement of the slide block, a certain amount of lubricant or grease needs to be applied to the slide block and/or guide rod for some pump body devices. However, the excess lubricant or grease will fall into other structures of the pump body device during the slide process of the slide block. Some pump body devices employ slide blocks which are made of plastic, which meet the slide requirements through the self-lubrication of the slide block. Although this kind of slide block does not need to be coated with lubricant or grease, however its guide accuracy is relatively poor.

In order to solve the above problems, please refer to FIG. 4. The slide assembly 12 of this embodiment also includes an oil-containing sleeve 123 which is arranged at the slide block 12'. The slide block 12' is slidably connected with the guide rod 121' through the oil-containing sleeve 123. In other words, the first installation hole 122'a and the second installation hole 122'b of this embodiment are respectively provided with one oil-containing sleeve 123, and the two guide rods 121' penetrate into the corresponding oil-containing sleeve 123, which is slidably connected with the guide rod 121'. At the same time, in order to ensure that the slide block 122' which penetrates through the guide rod 121', has a certain adjustment allowance, when installing the oil-containing sleeve 123, one oil-containing sleeve 123 is in interference fit with the first installation hole 122'a, while the oil-containing sleeve 123, which is installed in the second installation hole 122'b, can release a degree of freedom between the two guide rods 121', and further can release a degree of freedom in the direction which is perpendicular to the guide rod 121'. That is, the oil-containing sleeve 123, which is arranged inside the second installation hole 122'b, can deviate along the radial direction of the guide rod 121' in the second installation hole 122'b, but will not fall out from the second installation hole 122'b. Since the oil-bearing sleeve 123 has good self-lubricating property and high guide accuracy, there is no need to use lubricant or grease between the slide block 122' and the guide rod 121' to ensure the smooth movement of the slide block 122', which can not only prevent the lubricant or grease from falling into other structures of the pump body device 10 and polluting the pump body device 10, but also ensure that the guide accuracy of the slide block 122' can meet the use requirements of the injection pump 100. In addition, referring to FIG. 1, one oil-containing sleeve 123 is actually arranged inside the position-limiting hole 11d of this embodiment, and the hollow shaft 13' penetrates through the oil-containing sleeve 123, which is inside the position-limiting hole 11d, and is slidable relative to the oil-containing sleeve 123, so as to improve the stability of the movement of the hollow shaft 13'. It can be understood that in other embodiments, the oil-containing sleeve 123 may not be provided in the first installation hole 122'a, the second installation hole 122'b and the position-limiting hole 11d.

Please continue to refer to FIGS. 1 to 3 and 8 to 11, the pump body device 10 of this embodiment includes a displacement monitoring element 18 and a first circuit board 17. The first circuit board 17 is arranged at one end of the base body 11, which is adjacent to the push-pull box assembly 20, and is connected with the side wall of the base body 11, which is away from the accommodation cavity 11a. The displacement monitoring element 18 includes a first potentiometer 181 and a plunger assembly 182, which is connected with the second slide member 122 and has a contact head 1821. The first potentiometer 181 contacts the contact head 1821 to monitor the displacement of the second slide member 122.

Specifically, the first potentiometer 181 of this embodiment is a strip-shaped elongated potentiometer 181', which includes a first section 1811', a second section 1812' which is connected with the first section 1811', and a resistor 1813' which is arranged at the first section 1811' along the length direction of the elongated potentiometer 181'. The first section 1811 'is arranged inside the accommodation cavity 11a and is connected with the side wall of the base body 11 at the accommodation cavity 11a. The second section 1812' extends out of the accommodation cavity 11a from one end of the base body 11, which is provided with the first circuit board 17. The second section 1812' bends to the side, which is provided with the first circuit board 17, and is electrically connected with the first circuit board 17. The contact head 1821 contacts the resistor 1813' to monitor the displacement of the second slide member 122.

In related technologies, slide-line monitoring is mostly operable to monitor the displacement of the slide block. This monitoring method usually requires the use of linear displacement sensor. Because the volume of the linear displacement sensor is relatively large, the linear displacement sensor also needs to occupy more installation space.

The strip-shaped elongated potentiometer 181' is adopted in this embodiment. During the slide process of the slide block 122', the plunger assembly 182, which is installed at the slide block 122', slides along with the slide block 122', and the contact head 1821 of the plunger assembly 182 is always in contact with the resistor 1813' of the elongated potentiometer 181'. According to the different contact positions between the contact head 1821 and the resistor 1813', the elongated potentiometer 181' can transmit the corresponding monitoring signal to the first circuit board 17, thus monitoring the position of the slide block 122'. Since the strip-shaped elongated potentiometer 181' has simple structure and small volume and does not need to occupy too much installation space, the overall structure of the pump body device 10 of this embodiment can be more compact.

The resistor 1813' of this embodiment is made of self-lubricating material POM (polyoxymethylene), so that the contact head 1821 can slide along the resistor 1813', and the elongated potentiometer 181' can be made of flexible material, so as to bend the second section 1812' of the elongated potentiometer 181' during assembly, or can be directly prefabricated into the shape shown in FIG. 11.

Referring to FIGS 8 to 11, the plunger assembly 182 of this embodiment is mainly composed of a contact head 1821, a main body 1822, a positioning column 1822a, an installation groove 1822b, a spring 1823 and an engaging element 1824. The main body 1822 is formed with a positioning column 1822a, an installation groove 1822b, an abutting joint 1822c and a screw hole 1822d. The abutting joint 1822c is arranged along the radial direction of the installation groove 1822b and communicated with the installation groove 1822b. The axis of the screw hole 1822d is parallel to the axis of the positioning column 1822a, the engaging element 1824 is formed with a through hole 1824a and two clamping feet 1824a which are respectively arranged on both sides of the axis of the through hole 1824a. When assembling the plunger assembly 182, firstly the contact head 1821 is inserted into the installation groove1822b, a part structure of the contact head 1821 (i.e. the end which is in contact with the resistor 1813') penetrates through the installation groove1822b and extends out of the installation groove1822b, then a part structure of the spring 1823 is inserted into the installation groove 1822b and sheathed outside the contact head 1821, the other part structure of the spring 1823 is located outside the installation groove1822b, and finally the two clamping feet 1824a of the engaging element 1824 are faced towards the installation groove1822b, and the engaging element 1824 is inserted into the installation groove 1822b, and the structure of the spring 1823, which is outside the installation groove 1822b penetrates through the through hole 1824b at the engaging element 1824. By rotating the engaging element 1824, the two clamping feet 1824a of the engaging element 1824 abut against the side wall of the main body 1822 at the abutting joint 1822c. When disassembling the plunger assembly 182, just rotate the engaging element 1824 to the non-abutting position, and take out the engaging element 1824, spring 1823 and contact head 1821 from the installation groove1822b. When the plunger assembly 182 needs to be installed to the slide block 122', the positioning column 1822a of the main body 1822 is inserted into and matched with the positioning groove (not shown) which is formed at the slide block 122'; the end of the spring 1823, which is outside the installation groove 1822b, abuts against the slide block 122'; the first screw hole 1822d is aligned with the second screw hole (not shown); which is formed on the slide block 122'; and a screw is screwed into the first screw hole 1822d and the second screw hole; thus fastening the plunger assembly 182 to the slide block 122'. That is, the plunger assembly 182 of this embodiment is a detachable structure, and the plunger assembly 182 and the slide block 122' are detachably connected together. Therefore, it can facilitate the disassembly and assembly of the plunger assembly 182 when the plunger assembly 182 needs to be replaced, or it can avoid the use of tools when the members of the plunger assembly 182 are damaged, and the corresponding members can be replaced quickly by hand.

Referring to FIGS. 1 to 3 and 7, the pump body device 10 of this embodiment also includes an optocoupler 16, which is electrically connected with the first circuit board 17. The optocoupler 16 is mainly operable to detect the starting position of the push-pull box assembly 20 and transmit the detection signal to the first circuit board 17.

In related technologies, a plurality of circuit boards are usually arranged inside the accommodation cavity of the base body, the detection element for detecting a start position of the push-pull box assembly is electrically connected with one circuit board, and the displacement monitoring element for monitoring the displacement of the second slide member is electrically connected with another circuit board. Because there are many circuit boards which are arranged inside the accommodation cavity, these circuit boards will occupy more installation space inside the accommodation cavity, while the displacement monitoring element 18 and the optocoupler 16 of this embodiment share the same first circuit board 17, and the first circuit board 17 is arranged at the outer wall of the base body 11 rather than inside the accommodation cavity 11a. Therefore, this arrangement method will not occupy the installation space of the accommodation cavity 11a while reducing the total number of circuit boards, so as to ensure that the overall structure of the pump body device 10 can be more compact.

In addition, since one end of the guide rod 121' of this embodiment is fixed inside the first positioning hole 11b by inserting, and the first circuit board 17 is actually arranged at the side wall of the base body 11 and cover the first positioning hole 11b at the side which is away from the accommodation cavity 11a, such that the first circuit board 17 can stop and positionally limit the guide rod 121' when the first positioning hole 11b fails due to accidents, thus preventing the normal use of the injection pump 100 from being affected by the guide rod 121' which slides out of the first positioning hole 11b during the use of the injection pump 100. It should be noted that the term "cover" described here refers to that the first circuit board 17 can block both of the two first positioning holes 11b from the direction which faces the first circuit board 17, that is, along the axial direction of the guide rod 121', the projection of the two guide rods 121' is within the projection range of the first circuit board 17, but there can be a certain gap between the first circuit board 17 and the first positioning hole 11b.

Referring to FIGS. 1 to 3 and 12, the transmission assembly 14 of this embodiment also includes a gear mechanism 143 which is in drive connection with the drive mechanism 15, and the screw rod 141 is fixedly connected with the gear mechanism 143.

Specifically, the gear mechanism 143 of this embodiment is a pulley mechanism 143', which includes a synchronous belt 1431', a primary gear 1432 'and a secondary gear 1433'. The output shaft of the drive mechanism 15 is in drive connection with the primary gear 1432' through the synchronous belt 1431'. The primary gear 1432' is engaged with the secondary gear 1433', and the secondary gear 1433' is fixedly connected with the screw 141.

Specifically, in related technologies, most of the drive mechanism and screw rod need to be driven by a gear mechanism. The conventional gear mechanism is that the gear is directly in drive connection with the drive mechanism, that is, there is no synchronous belt, and the drive mechanism drives the screw rod to rotate through the gear mechanism. The arrangement of the gear mechanism can not only ensure that the torque output of the drive mechanism is amplified, but also ensure that the rotation speed of the screw rod meets the requirements. However, in the process of rapid loading, the drive mechanism needs to run at a high speed, while the conventional gear mechanism will not only bring huge noise in the process of rotation, but also sharply reduce the service life of the drive mechanism and the gear mechanism. In addition, this connection mode has no overload protection and is prone to accidents.

The pulley mechanism 143' of this embodiment is a two-stage transmission structure. The first stage is synchronous pulley transmission, that is, the output shaft of the drive mechanism 15 is in drive connection with the primary gear 1432' through the synchronous belt 1431', rather than the output shaft of the drive mechanism 15 is in direct drive connection with the primary gear 1432'. The second stage is a meshing transmission between the primary gear 1432' and the secondary gear 1433'. The structure of synchronous belt pulley can ensure the accurate transmission of the output revolutions of the drive mechanism 15. At the same time, the synchronous belt 1431' can provide overload protection for the drive mechanism 15, and the elasticity of the synchronous belt 1431' itself can also reduce the transmission noise. Therefore, the transmission assembly 14 of this embodiment not only has the function of noise reduction, but also improves the safety of the injection pump 100.

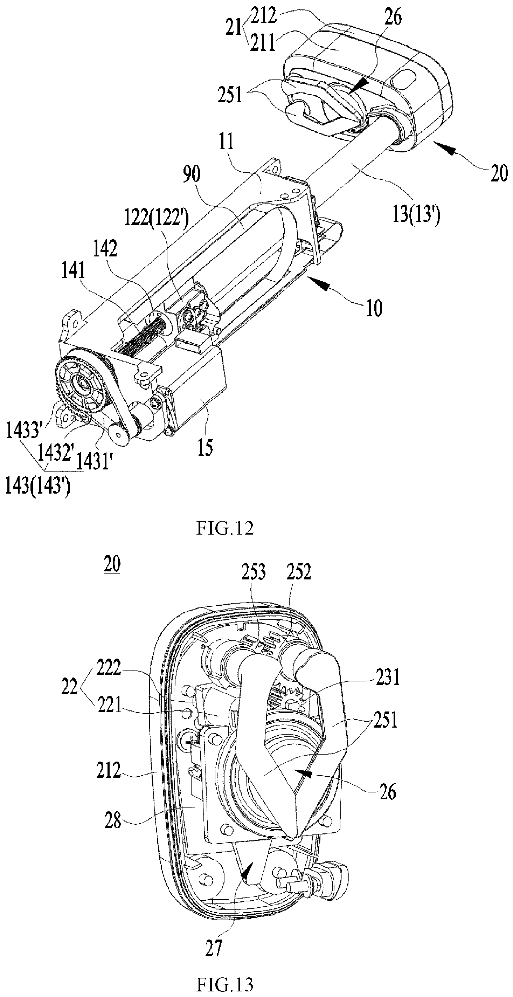

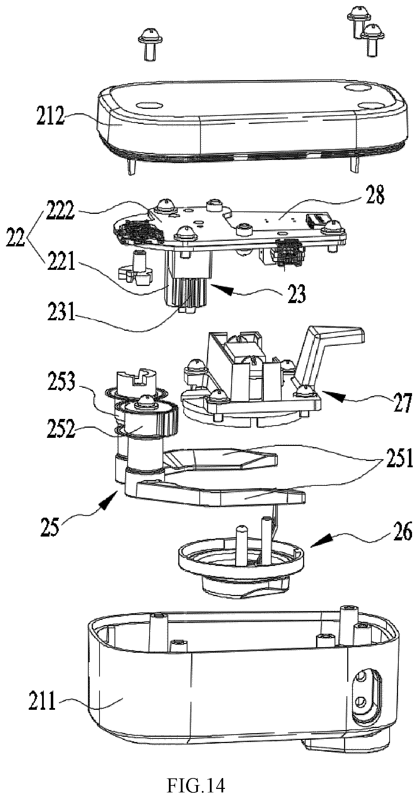

Referring to FIGS. 12 to 14, the push-pull box assembly 20 of this embodiment includes a box 21, a probe assembly 26, a pressure sensor assembly 27, a claw clamping mechanism 25 and a claw clamping drive assembly 22. The pressure sensor assembly 27 is arranged inside the box 21 and overlapped with the probe assembly 26. The claw clamping mechanism 25 has two clamping claws 251, which include a drive rod and a clamping part, wherein the clamping part is located outside the box 21. The drive rod is located at one end of the clamping part and penetrates through the box 21. The claw clamping drive assembly 22 is arranged inside the box 21. The claw clamping drive assembly includes a second circuit board 222, a push-pull box motor 221 which is arranged at the second circuit board 222, and a claw clamping transmission assembly 23 which is connected with the push-pull box motor 221 and the drive rod. The push-pull box motor 221 and the claw clamping transmission assembly 23 are located at one end of the second circuit board 222. A sensor installation space is formed between a suspended part and a clamping part of the second circuit board 222, and the probe assembly 26 and the pressure sensor assembly 27 are stacked inside the sensor installation space.

The injection pump 100 of this embodiment connects the push-pull box motor 221 and the drive rod through the claw clamping transmission assembly 23, which enables the claw clamping transmission assembly 23, the push-pull box motor 221 and the drive rod to form a solid structure, which is supported between the second circuit board 222 and the clamping part and is located at one end of second circuit board 222, while realizing the connection. In such a way, the part of the second circuit board 222, which is not provided with the push-pull box motor 221 and the claw clamping transmission assembly 23, is suspended relative to the clamping part, that is, it forms the suspended part of the second circuit board 222. A sensor installation space is formed between the suspended part and the clamping part of the second circuit board 222, and the probe assembly 26 and the pressure sensor assembly 27 are stacked inside the sensor installation space. Through the above arrangement, the space between the second circuit board 222 and the clamping part is reasonably used to avoid the probe assembly 26 and the pressure sensor assembly 27 occupying additional space in the push-pull box assembly 20, effectively improving the structural compactness of the internal parts of the push-pull box assembly 20 and reducing the overall volume of the push-pull box assembly 20.

In this embodiment, the box 21 includes a box body 211 and a box cover 212. The claw clamping mechanism 25, the pressure sensor assembly 27, the probe assembly 26 and the claw clamping drive assembly 22 are installed inside the box body 211 sequentially. The box cover 212 is covered on the box body 211 to facilitate the assembly of the push-pull box assembly 20. That is, the opening of the box body 211 faces the box cover 212. The claw clamping mechanism 25, the pressure sensor assembly 27, the probe assembly 26 and the claw clamping drive assembly 22 are installed inside the box body 211 in turn from the opening. After the box cover 212 is covered on the box body 211, the component which is closest to the box cover 212 among the claw clamping mechanism 25, the pressure sensor assembly 27, the probe assembly 26 and the claw clamping drive assembly 22 is the claw clamping drive assembly 22. The box body 211 and the box cover 212 are preferably detachably connected, such as through screw connection or engagement connection.

The claw clamping mechanism 25, the pressure sensor assembly 27, the probe assembly 26 and the claw clamping drive assembly 22 can also be assembled outside the box 21 and then installed inside the box 21 as a whole.

The push-pull box assembly 20 of this embodiment also includes a support plate 28 which is arranged inside the box 21, the box body 211 has a positioning support part which positionally limits and supports the support plate 28, and the second circuit board 222 is fixedly connected with the support plate 28. Through arranging the support plate 28, the support plate 28 can cooperate with the positioning support part of the box 21 to complete the position-limitation and support of the positioning support part to the support plate 28, and then complete the position-limitation effect of the second circuit board 222 inside the box 21. The second circuit board 222 can also be positionally limited and supported by the positioning support part of the box body 211 to achieve the position-limitation effect.

In this embodiment, the support plate 28 can be a metal sheet support, or can be made of other materials, or can be a control circuit board with control components corresponding to the probe assembly 26 and the pressure sensor assembly 27.

It can be understood that the support plate 28 is a plate-shaped structure for the sake of convenient processing and improving structural compactness. In order to facilitate installation, the projection surface of the support plate 28 covers the second circuit board 222. Therefore, the support plate 28 is matched with the positioning part of the box body 211.

Of course, the support plate 28 may not be provided, and the claw clamping drive assembly may be directly fixed to the support of the pressure sensor assembly 27 or to the inner wall of the box 21.

In order to ensure the structural stability of the installation space and avoid the second circuit board 222 from squeezing the probe assembly 26 and the pressure sensor assembly 27, the support plate 28 is located at the side of the second circuit board 222, which is away from the box cover 212. The support plate 28 is provided with a hollow avoidance part for the push-pull box motor 221 and the claw clamping drive assembly 23 to penetrate through. That is, the support plate 28 can provide a support force, which is away from the clamping part, for the second circuit board 222, and can effectively support the second circuit board 222 when the injection pump 100 falls or other sudden impact occurs, so as to ensure the connection stability between the claw clamping transmission assembly 23 with the push-pull box motor 221 and the drive rod, and avoid the component damage, which is caused by the extrusion to the probe assembly 26 and the pressure sensor assembly 27 of the second circuit board 222.

Further, the claw clamping mechanism 25 also includes a transmission gear 252 which drives the two clamping claws 251 to move oppositely. The transmission gear 252 is arranged at the end of the drive rod, which extends into the box body 211. The claw clamping transmission assembly 23 includes a drive gear 231 which is driven by the push-pull box motor 221, and the transmission gear 252 is engaged with the drive gear 231. The axial directions of the transmission gear 252 and the drive gear 231 are arranged along the installation direction of the claw clamping drive assembly toward the box body 211. When the claw clamping drive assembly 22 is installed in place relative to the box body 211, the transmission gear 252 is engaged with the drive gear 231. The drive gear 231 is rotated by the push-pull box motor 221, and the transmission gear 252 is engaged with the drive gear 231, such that the relative movement operation of the two clamping claws 251 can be realized under the driving of the drive gear 231. When the two clamping claws 251 approaches each other, the clamping of the piston handle of the injector 200 by the claw clamping mechanism 25 is completed. When the two clamping claws 251 are away from each other, the separation of the claw clamping mechanism 25 from the piston handle of the syringe 200 is completed.

For convenience of installation, the axial directions of the transmission gear 252 and the drive gear 231 are arranged along the installation direction of the claw clamping drive assembly 22 toward the box body 211. When the claw clamping drive assembly 22 is installed in place relative to the box body 211, the transmission gear 252 is engaged with the drive gear 231. That is, when the claw clamping drive assembly is installed into the box body 211, the drive gear 231 at the claw clamping drive assembly is directly engaged with the transmission gear 252 of the claw clamping mechanism 25 which has been already installed on the box body 211, thus facilitating assembly.

In this embodiment, the transmission gear 252 and the drive gear 231 are spur gears. The above arrangement facilitates the processing and assembly of the transmission gear 252 and the drive gear 231. When installing the claw clamping drive assembly, the axial directions of the transmission gear 252 and the drive gear 231 are in parallel, and the transmission gear 252 and the drive gear 231 are adjacent to each other, so that the straight teeth of the transmission gear 252 and the straight teeth of the drive gear 231 are directly inserted and engaged with each other, and the engagement between the straight teeth of the transmission gear 252 and the straight teeth of the drive gear 231 is completed. It should be noted that the claw clamping mechanism 25 of this embodiment also includes a driven gear 7 which is engaged with the transmission gear 252, and the driven gear 253 is also a spur gear.

In another embodiment, the transmission gear 252 and the drive gear 231 are bevel gears, and the large diameter end of the drive gear 231 faces the second circuit board 222. For the sake of convenient installation, it is preferable to face the large diameter end of the drive gear 231 towards the second circuit board 222, so that when the claw clamping drive assembly 22 is installed inside the box body 211, the small diameter end of the drive gear 231 first coincides with the transmission gear 252 in the axial direction, so as to facilitate the engagement connection of the transmission gear 252 and the drive gear 231. It can be understood that if the driven gear 253 is also provided in this embodiment, the driven gear 7 is also a bevel gear.

In addition, when the push-pull box motor 221 is powered off, the drive gear 231 of the present embodiment can rotate under the driving of an external force. In particular cases (such as emergency removal of syringe 200 or power failure), the push-pull box motor 221 can drive the drive gear 231 to rotate through the external force when the push-pull box motor 221 is powered off, so that the push-pull box motor 221 does not affect the rotation of the drive gear 231. The two clamping claws 251 can be directly and manually separated. In the process of manually separating the two clamping claws 251, the transmission gear 252 rotates and drives the drive gear 231 to rotate. That is, the clamping claw 251 reversely drives the transmission gear 252, the drive gear 231 and the push-pull box motor 221 to rotate, and the transmission gear 252 and the drive gear 231 will not be self-locked. Through the above arrangement, the clamping claw 251 can be separated directly and manually in particular cases to complete the separation and unlocking operation of the claw clamping mechanism 25 on the piston handle of the syringe 200, which is convenient for operation and capable of shortening the unlocking time. Moreover, no unlocking structure needs to be arranged separately, which effectively reduces the volume of the push-pull box assembly 20 and simplifies the structure of the injection pump 100.

Referring to FIGS. 4, 12 and 16, the hollow shaft 13' of the present embodiment includes a shaft body 131' which has an installation channel 13'a and a sleeve 132' which is sleeved inside the shaft body 131' and separates the installation channel 13'a into a first sub-channel 13'b and a second sub-channel 13'c. The injection pump 100 also includes a cable 90 and a main board 80 which is laid on the bottom housing 31. The screw rod **141** penetrates through the first sub-channel 13'b, the cable 90 penetrates through the second sub-channel 13'c, and the push-pull box assembly 20 is electrically connected with the main board 80 through the cable 90.

Specifically, during the use of the injection pump 100 of this embodiment, a corresponding signal needs to be transmitted between the push-pull box assembly 20 and the main board 80. For example, the main board 80 needs to transmit a control signal to the push-pull box motor 221 to control the opening and closing of the claw clamping mechanism 251. During the injection process, the pressure sensor assembly 27, which is arranged inside the box 21m can detect the pressure inside the barrel of the syringe 200 and transmit the detection signal to the main board 80. By electrically connecting the push-pull box assembly 20 with the main board 80 through the cable 90, the transmission of these control signals and detection signals can be realized. Since the hollow shaft 13' of this embodiment is sheathed outside the screw rod **141,** and the cable 90 penetrates through the hollow shaft 13', the hollow shaft 13 'of this embodiment is also provided with a sleeve 132', which is inserted inside the shaft 131', the screw rod **141** penetrates through the first sub-channel 13'b, and the cable 90 penetrates through the second sub-channel 13'c. That is, the cable 90 can be separated from the screw rod **141** through the sleeve 132', to prevent the screw rod **141** from damaging the cable 90 during high-speed rotation. Those skilled in the art should know that, the main board 80 can use various existing chips with signal input and signal output as control devices and can uses electrical signal control mode or software control mode for control. The cable 90 can be flexible flat cable (FFC) or other cables that can transmit electrical signals.

In addition, in related technologies, the main board is mostly vertically arranged inside the inner cavity, and some injection pumps are also provided with multiple divisional circuit boards outside the main board, which are stacked on a side of the main board. This arrangement method greatly occupies the installation space of the inner cavity.

In this embodiment, a large main board 80 is arranged and the main board 80 is laid on the bottom housing 31, and no other divisional circuit boards are arranged near the main board 80. Therefore, the installation space of the inner cavity 30a can be greatly saved, and the overall structure of the injection pump 100 can be more compact.

Referring FIGS.1-20, an injection pump 100 according to an embodiment of this disclosure includes a housing 30, a syringe installation assembly 600, a push-pull box assembly 20, a pump body device 10, a battery module 40, a power module 50 and a display screen assembly 70. The housing 30 includes a bottom housing 31 and a cover plate 32. The syringe installation assembly 600 includes an installation base 61 and a clamping shank 62, wherein the installation base 61, the bottom housing 31 and the cover plate 32 enclose an inner cavity 30a of the housing 30. A clamping channel 61a and a clamping shank installation space 61b, which is communicated with the clamping channel 61a from a side of the clamping channel 61a, are formed on a side wall of the installation base 61, which is away from the inner cavity 30a. The clamping shank installation space 61b is arranged at an end of the installation base 61 and communicated with outside. The clamping shank 62 is arranged inside the clamping shank installation space 61b, and the clamping shank 62 rotates relative to the installation base 61 to clamp or release the syringe 200. The push-pull box assembly 20 is arranged outside the housing 30 and faces an end of the clamping channel 61a, which is adjacent to the clamping shank 62. The pump body device 10 is arranged inside the inner cavity 30a and is located at a side of the inner cavity 30a, which is adjacent to the syringe installation assembly 600. The pump body device 10 includes a base body 11, and a slide assembly 12, a connection shaft 13, a transmission assembly 14 and a drive mechanism 15. Wherein the base body 11, and slide assembly 12, connection shaft 13, transmission assembly 14 and drive mechanism 15 are arranged at the base body 11. The base body 11 has an accommodation cavity 11a, which is located at a side of the base body 11, which is away from the syringe installation assembly 600. The slide assembly 12 includes two guide rods 121' which are arranged at intervals and in parallel inside the accommodation cavity 11a and a slide block 122' which is penetrated by the two guide rods 121'. The connection shaft 13 is a hollow shaft 13' which is arranged between the two guide rods 121', one end of the hollow shaft 13' is connected with the slide block 122', and the other end of the hollow shaft 13' which is away from the slide block 122' is located outside the housing 30 and connected with the push-pull box assembly 20. The drive mechanism 15 is arranged inside the accommodation cavity 11a and is located at a side of the accommodation cavity 11a which is away from the push-pull box assembly 20. The transmission assembly 14 includes a gear mechanism 143 which is in drive connection with the drive mechanism 15, and a screw rod 141 which is fixedly connected with the gear mechanism 143, wherein the screw rod 141 is in transmission connection with the slide assembly 12 and is inserted inside the hollow shaft. The battery module 40 is arranged inside the inner cavity 30a and located at an extension line of an output shaft 151 of the drive mechanism 15. The power module 50 is arranged inside the inner cavity 30a, along a transverse direction which is perpendicular to a length direction of the pump body device 10. The power module 50 and the battery module 40 are arranged side-by-side at the same side of the base body 11. The display screen assembly 70 is arranged at a side of the syringe installation assembly 600, which is away from the pump body device 10 and covers the syringe installation assembly 600.

The above embodiments only describe several preferable embodiments of this disclosure, but it cannot be understood as limiting the present disclosure. The invention is defined by the appended claims.

## Claims

1. An injection pump (100), which is used together with a syringe, comprising:
a housing (30), which comprises a bottom housing (31) and a cover plate (32);
a syringe installation assembly (60), wherein the syringe installation assembly (60), the bottom housing (31) and the cover plate (32) enclose an inner cavity (30a) of the housing (30); the syringe installation assembly (60) comprises an installation base (61) and a clamping shank (62), and the clamping shank (62) rotates relative to the installation base (61) to clamp or release the syringe;
a push-pull box assembly (20), which is arranged outside the housing (30) and faces an end of the syringe installation assembly (60);
a pump body device (10), which is arranged inside the inner cavity (30a) and is located at a side of the inner cavity (30a), which is adjacent to the syringe installation assembly (60); wherein the pump body device (10) comprises a base body (11), a slide assembly (12), a connection shaft (13), a transmission assembly (14) and a drive mechanism (15); wherein the slide assembly (12), the connection shaft (13), the transmission assembly (14) and the drive mechanism (15) are arranged at the base body (11); wherein one end of the connection shaft (13) is connected with the slide assembly (12), and the other end of the connection shaft (13), which is away from the slide assembly (12), is located outside the housing (30) and connected with the push-pull box assembly (20); wherein the drive mechanism (15) is arranged at a side of the base body (11), which is away from the syringe installation assembly (60), wherein the drive mechanism (15) is in drive connection with the transmission assembly (14), and the transmission assembly (14) is in transmission connection with the slide assembly (12);
a battery module (40), which is arranged inside the inner cavity (30a), wherein the battery module (40) is located at the same side of the base body (11) where the drive mechanism (15) is arranged;
a power module (50), which is arranged inside the inner cavity (30a), along a transverse direction which is perpendicular to a length direction of the pump body device (10); wherein the power module (50) is arranged at the same side of the base body (11) where the battery module (40) is located and the power module (50) is arranged side-by-side to the battery module (40), wherein one of the battery module (40) and the power module (50) is located along an extension line of an output shaft (151) of the drive mechanism (15); and
a display screen assembly (70), which is arranged at a side of the syringe installation assembly (60), which is away from the pump body device (10); wherein the display screen assembly (70) covers the clamping shank (62) of the syringe installation assembly (60).

2. The injection pump (100) according to claim **1, characterized in that**, the drive mechanism (15) is arranged at one end of the base body (11), which is away from the push-pull box assembly (20).

3. The injection pump (100) according to claim 1 or 2, **characterized in that**, the battery module (40) is located at the extension line of the output shaft (151) of the drive mechanism (15), the power module (50) is located at a side of the battery module (40), which is away from the pump body device (10).

4. The injection pump (100) according to claim 1 or 2, **characterized in that**, length directions of the battery module (40) and the power module (50) are both parallel to the length direction of the pump body device (10); and the transverse direction, which is perpendicular to the length direction of the pump body device (10), is parallel to thickness directions of the battery module (40) and the power module (50).

5. The injection pump (100) according to claim 1 or 2, **characterized in that**, the installation base (61), the bottom housing (31) and the cover plate (32) enclose the inner cavity (30a);
wherein a clamping channel (61a) and a clamping shank installation space (61b) are formed on a side wall of the installation base (61), which is away from the inner cavity (30a);
wherein the clamping shank installation space (61b) is communicated with the clamping channel (61a) from a side of the clamping channel (61a), and the clamping shank installation space (61b) is arranged at an end of the installation base (61), which is adjacent to the push-pull box assembly (20), and is communicated with outside;
wherein the clamping shank is arranged inside the clamping shank installation space.

6. The injection pump (100) according to claim 5, **characterized in that**, the clamping shank (62) comprises a connection part (621), a clamping part (622) and a pressure part (623), the syringe installation assembly (60) further comprises a rotation assembly (63), wherein the rotation assembly (63) comprises a clamping shank shaft (631), a power transmission slide block (632), a compression spring (633), a pin shaft (634) and a protective sleeve (635);
wherein the connection part (621) is connected with one end of the clamping shank shaft (631), and the other end of the clamping shank shaft (631) is rotatably connected with the installation base (61), wherein the power transmission slide block (632), the pin shaft (634) and at least a part of the clamping shank shaft (631) are arranged inside the installation base (61); wherein the power transmission slide block (632) is provided with a spiral groove (632a), and the installation base (61) positionally limits a rotation of the power transmission slide block (632); wherein the pin shaft (634) is connected with the clamping shank shaft (631) and slidably matched with the spiral groove (632a); wherein the compression spring (633) is sheathed outside the clamping shank shaft (631), and both ends of the compression spring (633) abut against the power transmission slide block (632) and the connection part (621), respectively; wherein the pin shaft (634) slides along the spiral groove (632a) to push the power transmission slide block (632) to move axially to compress or release the compression spring (633);
wherein both ends of the spiral groove (632a) are closed and a dead center is arranged at an end of the spiral groove (632a), which is away from the connection part (621);
wherein the protective sleeve (635) is detachably sheathed outside the power transmission slide block (632) and covers the spiral groove (632a), wherein the clamping shank shaft (631) is provided with an installation hole, and at least part of the pin shaft is installed inside the installation hole, and the protective sleeve (634) covers both ends of the pin shaft (634).

7. The injection pump (100) according to claim 1 or 2, **characterized in that**, the connection shaft (13) is a hollow shaft (13'), wherein the slide assembly (12) comprises a first slide member (121), which is arranged at the base body (11), and a second slide member (122), which is slidably connected with the first slide member (121), wherein the transmission assembly (14) comprises a screw rod (141), which is in drive connection with the second slide member (122);
wherein the screw rod (141) is arranged in parallel and at intervals with the first slide member (121) and is inserted inside the hollow shaft (13').

8. The injection pump (100) according to claim 7, **characterized in that**, the base body (11) has an accommodation cavity (11a), the slide assembly (12) is arranged inside the accommodation cavity (11a); wherein the pump body device (10) comprises a displacement monitoring element (18) and a first circuit board (17);
the first circuit board (17) is arranged at one end of the base body (11), which is adjacent to the push-pull box assembly (20), and is connected with a side wall of the base body (11), which is away from the accommodation cavity (11a);
the displacement monitoring element (18) comprises an elongated potentiometer (181), which is strip-shaped, and a plunger assembly (182), which is connected with the second slide member (122) and has a contact head (1821); wherein the elongated potentiometer (181) comprises a first section (1811'), a second section (1812') which is connected with the first section (1811'), and a resistor (1813') which is arranged at the first section (1811') along a length direction of the elongated potentiometer (181); wherein the first section (1811') is arranged inside the accommodation cavity (11a) and is connected with a side wall of the base body (11), which is positioned at the accommodation cavity (11a), the second section (1812') extends out of the accommodation cavity (11a) from said end of the base body (11), which is provided with the first circuit board (17), wherein the second section (1812') is bended to a side, where the first circuit board (17) is provided, and is electrically connected with the first circuit board (17); wherein the contact head (1821) contacts with the resistor (1813') to monitor a displacement of the second slide member (122).

9. The injection pump (100) according to claim 7, **characterized in that**, the hollow shaft (13') comprises a shaft body (131'), which has an installation channel (13'a) and a sleeve (132'), which is sleeved inside the shaft body (131') and separates the installation channel (13'a) into a first sub-channel (13'b) and a second sub-channel (13'c); wherein the injection pump (100) further comprises a cable (90) and a main board (80) which is laid on the bottom housing (31);
wherein the screw rod (141) penetrates through the first sub-channel (13'b);
the cable (90) penetrates through the second sub-channel (13'b), and the push-pull box assembly (20) is electrically connected with the main board (80) through the cable (90).

10. The injection pump (100) according to claim 1 or 2, **characterized in that**, the push-pull box assembly (20) includes a box (21), a probe assembly (26), a pressure sensor assembly (27), a claw clamping mechanism (25) and a claw clamping drive assembly (22);
the pressure sensor assembly (27) is arranged inside the box (21) and overlapped with the probe assembly (26);
the claw clamping mechanism (25) has two clamping claws (251), which comprise a drive rod and a clamping part, wherein the clamping part is located outside the box (21); wherein the drive rod is located at an end of the clamping part and penetrates through the box (21);
wherein the claw clamping drive assembly (22) is arranged inside the box body (211); wherein the claw clamping drive assembly (22) comprises a second circuit board (222), a push-pull box motor (221) which is arranged at the second circuit board (222), and a claw clamping transmission assembly (22) which is connected with the push-pull box motor (221) and the drive rod; wherein the push-pull box motor (221) and the claw clamping transmission assembly (23) are located at an end of the second circuit board (222); wherein a sensor installation space is formed between a suspended part and a clamping part of the second circuit board (222), and the probe assembly (26) and the pressure sensor assembly (27) are stacked inside the sensor installation space.

11. The injection pump (100) according to claim 10, **characterized in that**, the box (21) comprises a box body (211) and a box cover (212); wherein the claw clamping mechanism (25), the pressure sensor assembly (27), the probe assembly (26) and the claw clamping drive assembly (22) are installed inside the box body (211) sequentially, wherein the box cover (212) is covered on the box body (211);
wherein the claw clamping mechanism (25) further comprises a transmission gear (252) which drives the two clamping claws (251) to move oppositely, wherein the transmission gear (252) is arranged at an end of the drive rod, which extends into the box body (211);
wherein the claw clamping transmission assembly (23) comprises a drive gear (231) which is driven by the push-pull box motor (221), and the transmission gear (252) is engaged with the drive gear (231);
wherein axial directions of the transmission gear (252) and the drive gear (231) are arranged along an installation direction of the claw clamping drive assembly (22) toward the box body (211); wherein when the claw clamping drive assembly (22) is installed in place relative to the box body (211), the transmission gear (252) is engaged with the drive gear (231).

## Patentansprüche

1. Eine Injektionspumpe (100), die zusammen mit einer Spritze verwendet wird, umfassend:
ein Gehäuse (30), das ein unteres Gehäuse (31) und eine Abdeckplatte (32) umfasst;
eine Spritzeneinsetzvorrichtung (60), wobei die Spritzeneinsetzvorrichtung (60), das untere Gehäuse (31) und die Abdeckplatte (32) einen inneren Hohlraum (30a) des Gehäuses (30) umschließen, die Spritzeneinsetzvorrichtung (60) eine Einsetzbasis (61) und einen Klemmschaft (62) umfasst und der Klemmschaft (62) sich relativ zur Einsetzbasis (61) dreht, um die Spritze zu klemmen oder zu lösen;
eine Push-Pull-Box-Baugruppe (20), die außerhalb des Gehäuses (30) angeordnet ist und einem Ende der Spritzeneinsetzvorrichtung (60) zugewandt ist;
eine Pumpenkörpervorrichtung (10), die innerhalb des inneren Hohlraums (30a) angeordnet ist und sich an einer Seite des inneren Hohlraums (30a) befindet, die an die Spritzeneinsetzvorrichtung (60) angrenzt; wobei die Pumpenkörpervorrichtung (10) einen Grundkörper (11), eine Gleiteinheit (12), eine Verbindungswelle (13), eine Übertragungseinheit (14) und einen Antriebsmechanismus (15) umfasst; wobei die Gleiteinheit (12), die Verbindungswelle (13), die Übertragungseinheit (14) und der Antriebsmechanismus (15) am Grundkörper (11) angeordnet sind; wobei ein Ende der Verbindungswelle (13) mit der Gleiteinheit (12) verbunden ist und das andere Ende der Verbindungswelle (13), das von der Gleiteinheit (12) abgewandt ist, sich außerhalb des Gehäuses (30) befindet und mit der Push-Pull-Box-Baugruppe (20) verbunden ist; wobei der Antriebsmechanismus (15) an einer Seite des Grundkörpers (11) angeordnet ist, die von der Spritzeneinsetzvorrichtung (60) abgewandt ist, wobei der Antriebsmechanismus (15) in Antriebsverbindung mit der Übertragungseinheit (14) steht und die Übertragungseinheit (14) in Getriebeverbindung mit der Gleiteinheit (12) steht;
ein Batteriemodul (40), das innerhalb des inneren Hohlraums (30a) angeordnet ist, wobei sich das Batteriemodul (40) auf derselben Seite des Grundkörpers (11) befindet, auf der auch der Antriebsmechanismus (15) angeordnet ist;
ein Leistungsmodul (50), das innerhalb des inneren Hohlraums (30a) entlang einer Querrichtung angeordnet ist, die senkrecht zu einer Längsrichtung der Pumpenkörpervorrichtung (10) angeordnet ist; wobei das Leistungsmodul (50) auf derselben Seite des Grundkörpers (11) angeordnet ist, auf der sich das Batteriemodul (40) befindet, und das Leistungsmodul (50) seitlich neben dem Batteriemodul (40) angeordnet ist, wobei sich entweder das Batteriemodul (40) oder das Leistungsmodul (50) entlang einer Verlängerungslinie einer Abtriebswelle (151) des Antriebsmechanismus (15) befindet; und
eine Bildschirmbaugruppe (70), die an einer Seite der Spritzeneinsetzvorrichtung (60) angeordnet ist, die von der Pumpenkörpervorrichtung (10) entfernt ist; wobei die Bildschirmbaugruppe (70) den Klemmschaft (62) der Spritzeneinsetzvorrichtung (60) abdeckt.

2. Die Injektionspumpe (100) nach Anspruch 1, **dadurch gekennzeichnet, dass** der Antriebsmechanismus (15) an einem Ende des Grundkörpers (11) angeordnet ist, das von der Push-Pull-Box-Baugruppe (20) abgewandt ist.

3. Die Injektionspumpe (100) nach Anspruch 1 oder 2, **dadurch gekennzeichnet, dass** sich das Batteriemodul (40) an der Verlängerungslinie der Abtriebswelle (151) des Antriebsmechanismus (15) befindet und sich das Leistungsmodul (50) an einer Seite des Batteriemoduls (40) befindet, die von der Pumpenkörpervorrichtung (10) abgewandt ist.

4. Die Injektionspumpe (100) nach Anspruch 1 oder 2, **dadurch gekennzeichnet, dass** die Längsrichtungen des Batteriemoduls (40) und des Leistungsmoduls (50) beide parallel zur Längsrichtung der Pumpenkörpervorrichtung (10) verlaufen und die Querrichtung, die senkrecht zur Längsrichtung der Pumpenkörpervorrichtung (10) verläuft, parallel zu den Dickenrichtungen des Batteriemoduls (40) und des Leistungsmoduls (50) verläuft.

5. Die Injektionspumpe (100) nach Anspruch 1 oder 2, **dadurch gekennzeichnet, dass** die Einsetzbasis (61), das untere Gehäuse (31) und die Abdeckplatte (32) den inneren Hohlraum (30a) umschließen;
wobei ein Klemmkanal (61a) und ein Klemmschaft-Einbauraum (61b) an einer Seitenwand der Einsetzbasis (61) ausgebildet sind, die vom inneren Hohlraum (30a) abgewandt ist;
wobei der Klemmschaft-Einbauraum (61b) von einer Seite des Klemmkanals (61a) aus mit dem Klemmkanal (61a) in Verbindung steht und der Klemmschaft-Einbauraum (61b) an einem Ende der Einsetzbasis (61) angeordnet ist, das an die Push-Pull-Box-Baugruppe (20) angrenzt, und mit der Außenwelt in Verbindung steht;
wobei der Klemmschaft innerhalb des Klemmschaft-Einbauraums angeordnet ist.

6. Die Injektionspumpe (100) nach Anspruch 5, **dadurch gekennzeichnet, dass** der Klemmschaft (62) ein Verbindungsteil (621), ein Klemmteil (622) und ein Druckteil (623) umfasst, die Spritzeneinsetzvorrichtung (60) ferner eine Drehvorrichtung (63) umfasst, wobei die Drehvorrichtung (63) eine Klemmschaftwelle (631), einen Kraftübertragungsgleitblock (632), eine Druckfeder (633), eine Stiftwelle (634) und eine Schutzhülse (635) umfasst;
wobei das Verbindungsteil (621) mit einem Ende der Klemmschaftwelle (631) verbunden ist und das andere Ende der Klemmschaftwelle (631) drehbar mit der Einsetzbasis (61) verbunden ist, wobei der Kraftübertragungsgleitblock (632), die Stiftwelle (634) und mindestens ein Teil der Klemmschaftwelle (631) innerhalb der Einsetzbasis (61) angeordnet sind; wobei der Kraftübertragungsgleitblock (632) mit einer Spiralnut (632a) versehen ist und die Einsetzbasis (61) die
Drehung des Kraftübertragungsgleitblocks (632) in seiner Position begrenzt; wobei die Stiftwelle (634) mit der Klemmschaftwelle (631) verbunden ist und gleitend in die Spiralnut (632a) passt; wobei die Druckfeder (633) außerhalb der Klemmschaftwelle (631) angeordnet ist und beide Enden der Druckfeder (633) jeweils am Kraftübertragungsgleitblock (632) und am Verbindungsteil (621) anliegen; wobei die Stiftwelle (634) entlang der Spiralnut (632a) gleitet, um den Kraftübertragungsgleitblock (632) zu verschieben, damit er sich axial bewegt, um die Druckfeder (633) zusammenzudrücken oder zu entlasten;
wobei beide Enden der Spiralnut (632a) geschlossen sind und ein Totpunkt an einem Ende der Spiralnut (632a) angeordnet ist, das vom Verbindungsteil (621) abgewandt ist;
wobei die Schutzhülse (635) abnehmbar außerhalb des Kraftübertragungsgleitblocks (632) angebracht ist und die Spiralnut (632a) abdeckt, wobei die Klemmschaftwelle (631) mit einem Einbauloch versehen ist und mindestens ein Teil der Stiftwelle innerhalb des Einbaulochs angebracht ist und die Schutzhülse (635) beide Enden der Stiftwelle (634) abdeckt.

7. Die Injektionspumpe (100) nach Anspruch 1 oder 2, **dadurch gekennzeichnet, dass** die Verbindungswelle (13) eine Hohlwelle (13') ist, wobei die Gleiteinheit (12) ein erstes Gleitelement (121), das am Grundkörper (11) angeordnet ist, und ein zweites Gleitelement (122) umfasst, das gleitend mit dem ersten Gleitelement (121) verbunden ist, wobei die Übertragungseinheit (14) eine Schraubstange (141) umfasst, die in Antriebsverbindung mit dem zweiten Gleitelement (122) steht;
wobei die Schraubstange (141) parallel und in Abständen zum ersten Gleitelement (121) angeordnet und in die Hohlwelle (13') eingesetzt ist.

8. Die Injektionspumpe (100) nach Anspruch 7, **dadurch gekennzeichnet, dass** der Grundkörper (11) einen Aufnahmeraum (11a) aufweist, wobei die Gleiteinheit (12) innerhalb des Aufnahmeraums (11a) angeordnet ist; wobei die Pumpenkörpervorrichtung (10) ein Verschiebungsüberwachungselement (18) und eine erste Leiterplatte (17) umfasst;
die erste Leiterplatte (17) ist an einem Ende des Grundkörpers (11) angeordnet, das an die Push-Pull-Box-Baugruppe (20) angrenzt, und mit einer Seitenwand des Grundkörpers (11) verbunden, die von dem Aufnahmeraum (11a) abgewandt ist;
das Verschiebungsüberwachungselement (18) umfasst ein längliches Schiebepotentiometer (181) und eine Kolbenbaugruppe (182), die mit dem zweiten Gleitelement (122) verbunden ist und einen Kontaktkopf (1821) aufweist; wobei das längliche Potentiometer (181) einen ersten Abschnitt (1811') umfasst, einen zweiten Abschnitt (1812'), der mit dem ersten Abschnitt (1811') verbunden ist, und einen Widerstand (1813'), der am ersten Abschnitt (1811') entlang einer Längsrichtung des länglichen Potentiometers (181) angeordnet ist; wobei der erste Abschnitt (1811') innerhalb des Aufnahmeraums (11a) angeordnet ist und mit einer Seitenwand des Grundkörpers (11) verbunden ist, die am Aufnahmeraum (11a) positioniert ist, wobei sich der zweite Abschnitt (1812') aus dem Aufnahmeraum (11a) aus dem Ende des Grundkörpers (11) heraus erstreckt, der mit der ersten Leiterplatte (17) versehen ist, wobei der zweite Abschnitt (1812') zu einer Seite gebogen ist, wo die erste Leiterplatte (17) angeordnet ist, und elektrisch mit der ersten Leiterplatte (17) verbunden ist; wobei der Kontaktkopf (1821) mit dem Widerstand (1813') in Kontakt steht, um eine Verschiebung des zweiten Gleitelements (122) zu überwachen.

9. Die Injektionspumpe (100) nach Anspruch 7, **dadurch gekennzeichnet, dass** die Hohlwelle (13') einen Wellenkörper (131') umfasst, der einen Einbaukanal (13'a) und eine Hülse (132') aufweist, die in den Wellenkörper (131') eingeschoben ist und den Einbaukanal (13'a) in einen ersten Teilkanal (13'b) und einen zweiten Teilkanal (13'c) unterteilt; wobei die Injektionspumpe (100) ferner ein Kabel (90) und eine Hauptplatine (80) umfasst, die auf dem unteren Gehäuse (31) aufgelegt ist;
wobei die Schraubstange (141) durch den ersten Teilkanal (13'b) hindurchragt;
das Kabel (90) durch den zweiten Teilkanal (13'b) hindurchgeht und die Push-Pull-Box-Baugruppe (20) über das Kabel (90) elektrisch mit der Hauptplatine (80) verbunden ist.

10. Die Injektionspumpe (100) nach Anspruch 1 oder 2, **dadurch gekennzeichnet, dass** die Push-Pull-Box-Baugruppe (20) einen Kasten (21), eine Sondenbaugruppe (26), eine Drucksensorbaugruppe (27), einen Klauenklemmmechanismus (25) und eine Klauenklemmantriebsbaugruppe (22) umfasst;
die Drucksensorbaugruppe (27) innerhalb des Kastens (21) angeordnet ist und die Sondenbaugruppe (26) überlappt;
der Klauenklemmmechanismus (25) zwei Klemmklauen (251) aufweist, die eine Antriebsstange und ein Klemmteil umfassen, wobei sich das Klemmteil außerhalb des Kastens (21) befindet; wobei sich die Antriebsstange an einem Ende des Klemmteils befindet und durch den Kasten (21) hindurchragt;
wobei die Klauenklemmantriebsbaugruppe (22) innerhalb des Kastenkörpers (211) angeordnet ist; wobei die Klauenklemmantriebsbaugruppe (22) eine zweite Leiterplatte (222) umfasst, einen Push-Pull-Box-Motor (221), der an der zweiten Leiterplatte (222) angeordnet ist, und eine Klauenklemmgetriebeanordnung (22), die mit dem Push-Pull-Box-Motor (221) und der Antriebsstange verbunden ist; wobei sich der Push-Pull-Box-Motor (221) und die Klauenklemmgetriebeanordnung (23) an einem Ende der zweiten Leiterplatte (222) befinden; wobei ein Sensor-Einbauraum zwischen einem hängenden Teil und einem Klemmteil der zweiten Leiterplatte (222) ausgebildet ist und die Sondenbaugruppe (26) und die Drucksensorbaugruppe (27) innerhalb des Sensor-Einbauraums übereinander angeordnet sind.

11. Die Injektionspumpe (100) nach Anspruch 10, **dadurch gekennzeichnet, dass** der Kasten (21) einen Kastenkörper (211) und einen Kastendeckel (212) umfasst; wobei der Klauenklemmmechanismus (25), die Drucksensorbaugruppe (27), die Sondenbaugruppe (26) und die Klauenklemmgetriebeanordnung (22) innerhalb des Kastenkörpers (211) nacheinander eingebaut sind, wobei der Kastendeckel (212) auf dem Kastenkörper (211) angebracht ist;
wobei der Klauenklemmmechanismus (25) ferner ein Getriebe (252) umfasst, das die beiden Klemmklauen (251) antreibt, um sich entgegengesetzt zu bewegen, wobei das Getriebe (252) an einem Ende der Antriebsstange angeordnet ist, die sich in den Kastenkörper (211) erstreckt;
wobei die Klauenklemmgetriebeanordnung (23) ein Antriebszahnrad (231) umfasst, das vom Push-Pull-Box-Motor (221) angetrieben wird, und das Getriebe (252) mit dem Antriebszahnrad (231) in Eingriff steht;
wobei die axialen Richtungen des Getriebes (252) und des Antriebsrads (231) entlang einer Einbaurichtung der Klauenklemmgetriebeanordnung (22) in Richtung des Kastenkörpers (211) angeordnet sind; wobei, wenn die Klauenklemmgetriebeanordnung (22) relativ zum Kastenkörper (211) eingebaut ist, das Getriebe (252) mit dem Antriebsrad (231) in Eingriff steht.

## Revendications

1. Pompe d'injection (100), laquelle est utilisée conjointement avec une seringue, comprenant :
un logement (30), lequel comprend un logement inférieur (31) et une plaque de couvercle (32) ;
un ensemble d'installation de seringue (60), où l'ensemble d'installation de seringue (60), le logement inférieur (31) et la plaque de couvercle (32) délimitent une cavité intérieure (30a) du logement (30) ; l'ensemble d'installation de seringue (60) comprend une base d'installation (61) et une queue de fixation (62), et la queue de fixation (62) tourne par rapport à la base d'installation (61) pour fixer ou libérer la seringue ;
un ensemble de boîtier de poussée-traction (20), lequel est agencé à l'extérieur du logement (30) et fait face à une extrémité de l'ensemble d'installation de seringue (60) ;
un dispositif de corps de pompe (10), lequel est agencé à l'intérieur de la cavité intérieure (30a) et est situé au niveau d'un côté de la cavité intérieure (30a), lequel est adjacent à l'ensemble d'installation de seringue (60) ; où le dispositif de corps de pompe (10) comprend un corps de base (11), un ensemble de coulissement (12), une tige de liaison (13), un ensemble de transmission (14) et un mécanisme d'entraînement (15) ; où l'ensemble de coulissement (12), la tige de liaison (13), l'ensemble de transmission (14) et le mécanisme d'entraînement (15) sont agencés au niveau du corps de base (11) ; où une première extrémité de la tige de liaison (13) est reliée à l'ensemble de coulissement (12), et l'autre extrémité de la tige de liaison (13), laquelle est à l'opposé de l'ensemble de coulissement (12), est située à l'extérieur du logement (30) et reliée à l'ensemble de boîtier de poussée-traction (20) ; où le mécanisme d'entraînement (15) est agencé au niveau d'un côté du corps de base (11), lequel est à l'opposé de l'ensemble d'installation de seringue (60), où le mécanisme d'entraînement (15) est en liaison d'entraînement avec l'ensemble de transmission (14), et l'ensemble de transmission (14) est en liaison de transmission avec l'ensemble de coulissement (12) ;
un module de batterie (40), lequel est agencé à l'intérieur de la cavité intérieure (30a), où le module de batterie (40) est situé au niveau du même côté du corps de base (11) où est agencé le mécanisme d'entraînement (15) ;
un module d'alimentation électrique (50), lequel est agencé à l'intérieur de la cavité intérieure (30a), le long d'une direction transversale laquelle est perpendiculaire à une direction longitudinale du dispositif de corps de pompe (10) ; où le module d'alimentation électrique (50) est agencé au niveau du même côté du corps de base (11) où est situé le module de batterie (40) et le module d'alimentation électrique (50) est agencé côte à côte du module de batterie (40), où l'un du module de batterie (40) et du module d'alimentation électrique (50) est situé le long d'une droite d'extension d'une tige de sortie (151) du mécanisme d'entraînement (15) ; et
un ensemble d'écran d'affichage (70), lequel est agencé au niveau d'un côté de l'ensemble d'installation de seringue (60), lequel est à l'écart du dispositif de corps de pompe (10) ; où l'ensemble d'écran d'affichage (70) couvre la queue de fixation (62) de l'ensemble d'installation de seringue (60).

2. Pompe d'injection (100) selon la revendication 1, **caractérisée en ce que**, le mécanisme d'entraînement (15) est agencé au niveau d'une première extrémité du corps de base (11), laquelle est à l'opposé de l'ensemble de boîtier de poussée-traction (20).

3. Pompe d'injection (100) selon la revendication 1 ou 2, **caractérisée en ce que**, le module de batterie (40) est situé au niveau de la droite d'extension de la tige de sortie (151) du mécanisme d'entraînement (15), le module d'alimentation électrique (50) est situé au niveau d'un côté du module de batterie (40), lequel est à l'écart du dispositif de corps de pompe (10).

4. Pompe d'injection (100) selon la revendication 1 ou 2, **caractérisée en ce que**, les directions longitudinales du module de batterie (40) et du module d'alimentation électrique (50) sont toutes deux parallèles à la direction longitudinale du dispositif de corps de pompe (10) ; et la direction transversale, laquelle est perpendiculaire à la direction longitudinale du dispositif de corps de pompe (10), est parallèle aux directions d'épaisseur du module de batterie (40) et du module d'alimentation électrique (50).

5. Pompe d'injection (100) selon la revendication 1 ou 2, **caractérisée en ce que**, la base d'installation (61), le logement inférieur (31) et la plaque de couvercle (32) délimitent la cavité intérieure (30a) ;
où un canal de fixation (61a) et un espace d'installation de queue de fixation (61b) sont formés sur une paroi latérale de la base d'installation (61), laquelle est à l'opposé de la cavité intérieure (30a) ;
où l'espace d'installation de queue de fixation (61b) communique avec la canal de fixation (61a) depuis un côté du canal de fixation (61a), et l'espace d'installation de queue de fixation (61b) est agencé au niveau d'une extrémité de la base d'installation (61), laquelle est adjacente à l'ensemble de boîtier de poussée-traction (20), et communique avec l'extérieur ;
où la queue de fixation est agencée à l'intérieur de l'espace d'installation de queue de fixation.

6. Pompe d'injection (100) selon la revendication 5, **caractérisée en ce que**, la queue de fixation (62) comprend une partie de liaison (621), une partie de fixation (622) et une partie de pression (623), l'ensemble d'installation de seringue (60) comprend en outre un ensemble de rotation (63), où l'ensemble de rotation (63) comprend une tige pour queue de fixation (631), un bloc de coulissement de transmission de puissance (632), un ressort de compression (633), une tige de broche (634) et un manchon protecteur (635) ;
où la partie de liaison (621) est reliée à une première extrémité de la tige pour queue de fixation (631), et l'autre extrémité de la tige pour queue de fixation (631) est reliée avec possibilité de rotation à la base d'installation (61), où le bloc de coulissement de transmission de puissance (632), la tige de broche (634) et au moins une partie de la tige pour queue de fixation (631) sont agencés à l'intérieur de la base d'installation (61) ; où le bloc de coulissement de transmission de puissance (632) est doté d'une rainure en spirale (632a), et la base d'installation (61) limite positionnellement une rotation du bloc de coulissement de transmission de puissance (632) ; où la tige de broche (634) est reliée à la tige pour queue de fixation (631) et est couplée avec possibilité de coulissement à la rainure en spirale (632a) ; où le ressort de compression (633) est emmanché à l'extérieur de la tige pour queue de fixation (631), et les deux extrémités du ressort de compression (633) butent contre le bloc de coulissement de transmission de puissance (632) et la partie de liaison (621), respectivement ; où la tige de broche (634) coulisse le long de la rainure en spirale (632a) pour pousser le bloc de coulissement de transmission de puissance (632) pour le déplacer axialement afin de comprimer ou de libérer le ressort de compression (633) ;
où les deux extrémités de la rainure en spirale (632a) sont fermées et un point mort est agencé au niveau d'une extrémité de la rainure en spirale (632a), lequel est à l'opposé de la partie de liaison (621) ;
où le manchon protecteur (635) est emmanché de manière amovible à l'extérieur du bloc de coulissement de transmission de puissance (632) et couvre la rainure en spirale (632a), où la tige pour queue de fixation (631) est dotée d'un trou d'installation, et au moins une partie de la tige de broche est installée à l'intérieur du trou d'installation, et le manchon protecteur (634) couvre les deux extrémités de la tige de broche (634).

7. Pompe d'injection (100) selon la revendication 1 ou 2, **caractérisée en ce que**, la tige de liaison (13) est une tige creuse (13'), où l'ensemble de coulissement (12) comprend un premier élément de coulissement (121), lequel est agencé au niveau du corps de base (11), et un second élément de coulissement (122), lequel est relié avec possibilité de coulissement au premier élément de coulissement (121), où l'ensemble de transmission (14) comprend une tige filetée (141), laquelle est en liaison d'entraînement avec le second élément de coulissement (122) ;
où la tige filetée (141) est agencée parallèlement et à intervalles avec le premier élément de coulissement (121) et est insérée à l'intérieur de la tige creuse (13').

8. Pompe d'injection (100) selon la revendication 7, **caractérisée en ce que**, le corps de base (11) présente une cavité de réception (11a), l'ensemble de coulissement (12) est agencé à l'intérieur de la cavité de réception (11a) ; où le dispositif de corps de pompe (10) comprend un élément de surveillance de déplacement (18) et une première carte de circuit imprimé (17) ;
la première carte de circuit imprimé (17) est agencée au niveau d'une première extrémité du corps de base (11), laquelle est adjacente à l'ensemble de boîtier de poussée-traction (20), et est reliée à une paroi latérale du corps de base (11), laquelle est à l'opposé de la cavité de réception (11a) ;
l'élément de surveillance de déplacement (18) comprend un potentiomètre allongé (181), lequel est en forme de bande, et un ensemble de plongeur (182), lequel est relié au second élément de coulissement (122) et présente une tête de contact (1821) ; où le potentiomètre allongé (181) comprend une première section (1811'), une seconde section (1812') laquelle est reliée à la première section (1811'), et une résistance (1813') laquelle est agencée au niveau de la première section (1811') le long d'une direction longitudinale du potentiomètre allongé (181) ; où la première section (1811') est agencée à l'intérieur de la cavité de réception (11a) et est reliée à une paroi latérale du corps de base (11), laquelle est positionnée au niveau de la cavité de réception (11a), la seconde section (1812') s'étend en dehors de la cavité de réception (11a) depuis ladite extrémité du corps de base (11), laquelle est dotée de la première carte de circuit imprimé (17), où la seconde section (1812') est courbée vers un côté, où est prévue la première carte de circuit imprimé (17), et est électriquement reliée à la première carte de circuit imprimé (17) ; où la tête de contact (1821) est en contact avec la résistance (1813') pour surveiller un déplacement du second élément de coulissement (122).

9. Pompe d'injection (100) selon la revendication 7, **caractérisée en ce que**, la tige creuse (13') comprend un corps de tige (131), lequel présente un canal d'installation (13'a) et un manchon (132'), lequel est emmanché à l'intérieur du corps de tige (131') et sépare le canal d'installation (13'a) en un premier sous-canal (13'b) et un second sous-canal (13'c) ; où la pompe d'injection (100) comprend en outre un câble (90) et une carte mère (80) laquelle est installée sur le logement inférieur (31) ;
où la tige filetée (141) pénètre à travers le premier sous-canal (13'b) ;
le câble (90) pénètre à travers le second sous-canal (13'c), et l'ensemble de boîtier de poussée-traction (20) est électriquement relié à la carte mère (80) par le biais du câble (90).

10. Pompe d'injection (100) selon la revendication 1 ou 2, **caractérisée en ce que**, l'ensemble de boîtier de poussée-traction (20) inclut un boîtier (21), un ensemble de sonde (26), un ensemble de capteur de pression (27), un mécanisme de fixation à griffes (25) et un ensemble d'entraînement de fixation à griffes (22) ;
l'ensemble de capteur de pression (27) est agencé à l'intérieur du boîtier (21) et est recouvert par l'ensemble de sonde (26) ;
le mécanisme de fixation à griffes (25) présente deux griffes de fixation (251), lesquelles comprennent une tige d'entraînement et une partie de fixation, où la partie de fixation est située à l'extérieur du boîtier (21) ; où la tige d'entraînement est située au niveau d'une extrémité de la partie de fixation et pénètre à travers le boîtier (21) ;
où l'ensemble d'entraînement de fixation à griffes (22) est agencé à l'intérieur du corps de boîtier (211) ; où l'ensemble d'entraînement de fixation à griffes (22) comprend une seconde carte de circuit imprimé (222), un moteur de boîtier de poussée-traction (221) lequel est agencé au niveau de la seconde carte de circuit imprimé (222), et un ensemble de transmission de fixation à griffes (22) lequel est relié au moteur de boîtier de poussée-traction (221) et à la tige d'entraînement ; où le moteur de boîtier de poussée-traction (221) et l'ensemble de transmission de fixation à griffes (23) sont situés au niveau d'une extrémité de la seconde carte de circuit imprimé (222) ; où un espace d'installation de capteur est formé entre une partie suspendue et une partie de fixation de la seconde carte de circuit imprimé (222), et l'ensemble de sonde (26) et l'ensemble de capteur de pression (27) sont empilés à l'intérieur de l'espace d'installation de capteur.

11. Pompe d'injection (100) selon la revendication 10, **caractérisée en ce que**, le boîtier (21) comprend un corps de boîtier (211) et un couvercle de boîtier (212) ; où le mécanisme de fixation à griffes (25), l'ensemble de capteur de pression (27), l'ensemble de sonde (26) et l'ensemble d'entraînement de fixation à griffes (22) sont installés à l'intérieur du corps de boîtier (211) successivement, où le couvercle de boîtier (212) est placé sur le corps de boîtier (211) et le couvre ;
où le mécanisme de fixation à griffes (25) comprend en outre un engrenage de transmission (252) lequel entraîne les deux griffes de fixation (251) pour les déplacer de manière opposée, où l'engrenage de transmission (252) est agencé au niveau d'une extrémité de la tige d'entraînement, laquelle s'étend dans le corps de boîtier (211) ;
où l'ensemble de transmission de fixation à griffes (23) comprend un engrenage d'entraînement (231) lequel est entraîné par le moteur de boîtier de poussée-traction (221), et l'engrenage de transmission (252) est engagé avec l'engrenage d'entraînement (231) ;
où les directions axiales de l'engrenage de transmission (252) et de l'engrenage d'entraînement (231) sont agencées le long d'une direction d'installation de l'ensemble d'entraînement de fixation à griffes (22) vers le corps de boitier (211) ; où lorsque l'ensemble d'entraînement de fixation à griffes (22) est en place par rapport au corps de boîtier (211), l'engrenage de transmission (252) est engagé avec l'engrenage d'entraînement (231).
